# EUROPEAN PATENT SPECIFICATION

(11) **EP 3 064 213 B1**
(45) Date of publication and mention of the grant of the patent: **19.08.2026**
(21) Application number: 14858535.9
(22) Date of filing: 28.10.2014
(51) Int. Cl.: A61K 38/00, A61K 9/08, A61K 9/10, A61K 38/22, A61K 38/43, A61K 39/395, A61K 47/12, A61K 47/34, C07K 1/00, C07K 14/52, C07K 14/575, C07K 16/00, C07K 19/00, C12N 9/00, A61P 43/00, A61P 5/00, A61K 38/50

(54) **PROTEIN AQUEOUS SUSPENSION**
WÄSSRIGE PROTEINSUSPENSION
SUSPENSION AQUEUSE DE PROTÉINE

(30) Priority: 28.10.2013 JP 2013223775
(43) Date of publication of application: 07.09.2016
(73) Proprietor: Terumo Kabushiki Kaisha, Tokyo 151-0072 (JP)
(72) Inventor: IZAKI Shunsuke, Takasaki-shi, Gunma, 370-0074 (JP); KIMOTO Tomoaki, Ashigarakami-gun Kanagawa 259-0151 (JP); HANDA Kenji, Ashigarakami-gun Kanagawa 259-0151 (JP); MIEDA Shiuhei, Ashigarakami-gun Kanagawa 259-0151 (JP); SHIRAKI Kentaro, Tsukuba-shi Ibaraki 305-8577 (JP); KURINOMARU Takaaki, Tsukuba-shi Ibaraki 305-8577 (JP); MARUYAMA Takuya, Tennodai, Tsukuba Ibaraki 305-8573 (JP)
(74) Representative: Casalonga
(86) International application number: PCT/JP2014/078667
(87) International publication number: WO 2015/064591

(56) References cited:
- WO-A1-2004/060920
- WO-A2-2007/076131
- WO-A2-2008/076819
- JP-A- 2002 530 323
- JP-A- 2003 514 844
- JP-A- 2004 510 753
- JP-A- 2005 506 956
- JP-A- 2006 503 124
- JP-A- 2006 512 416
- JP-A- 2006 523 609
- JP-A- 2009 539 810
- HU , TC ET AL.: "High-molecular-weight polymers for protein crystallization: poly-gamma- glutamic acid-based precipitants", ACTA CRYSTALLOGR D BIOL CRYSTALLOGR, vol. 64, no. 9, 2008, pages 957 - 63, XP055339599
- TAKUYA MARUYAMA ET AL.: "Tanpakushitsu Seizai no Konodoka o Misueta Polyamino San ni yoru Chinden/Saiyokaiho no Kaihatsu", PROTEIN SCIENCE SOCIETY OF JAPAN NENKAI PROGRAM YOSHISHU, vol. 14, 26 May 2014 (2014-05-26), pages 105, XP008184006

## Description

### Technical Field

The present invention relates to a method of stabilizing a protein, resulting in an aqueous suspension preparation of a complex of a protein and a polyamino acid wherein in the complex, the protein has at least one of shaking stress resistance, fluidity enhancement, oxidation resistance, thermal stability, and aggregation inhibitory properties and its activity is not deteriorated when concentrated. In addition, the complex may be re-dissolved in a buffer to obtain an aqueous liquid.

### Background Art

Owing to the progress of genetic recombination technique, there has been an increasing number of chances that a variety of proteins are provided as drugs. Particularly, there has been a remarkable technological progress concerning antibodies, where chimera antibodies, humanized antibodies and, further, antibodies based on application of sugar chain alteration technique have been put to practical use, and a number of antibody drugs have produced great therapeutic effects. While protein drugs were conventionally provided as freeze-dried preparations to be dissolved when put to use, they have been coming in recent years to be provided as aqueous preparations which can be used more easily. As for administration, besides, protein drugs were conventionally administered generally by intravenous injection, but an increasing number of protein drugs have been coming to be administered by subcutaneous injection, taking the patient's convenience into account. In subcutaneous injection, the dose volume must be reduced as compared to intravenous injection, so that it is indispensable to raise the concentration of the protein. In addition, particularly antibody drugs are used in large doses, so that a more rise in concentration is required in the case of subcutaneous injection of an antibody drug. Further, in recent years, attempts have been made to enhance the concentration for the purpose of reducing the administration frequency, and commercial pharmaceutical preparations having been provided include those which have a protein concentration of not less than 100 mg/mL.

However, proteins, which are biopolymers, are intrinsically vulnerable to physical stresses such as shaking and chemical stresses such as oxidation; particularly, proteins are more susceptible to denaturation when provided as aqueous preparations as compared to freeze-dried preparations. The denatured proteins often lose their original activity and form aggregates or association products, the reaction being often irreversible. Such formation of aggregates is more likely to occur as the protein concentration is higher, and the possibility of aggregate formation increases particularly during long-time storage and during transportation, because of an increase in the number of chances of exposure to a gas-liquid interface and interaction between protein molecules due to shaking or the like. The formation of aggregates lowers the efficacy of the protein drugs and, further, threatens the safety of the protein drugs. Specifically, the formation of aggregates has possibility of inducing an undesired immunoreaction such as production of an anti-drug antibody after administration (Non-patent Document 1). For this reason, the FDA in the United States and the EMEA in Europe have set guidelines as to evaluation of immunogenicity and stimulated countermeasures. Besides, among low-concentration preparations of proteins such as enzymes and cytokines, there are those which are liable to form aggregates, depending on the kind or preparation thereof, and not a few of them demand care in handling at the time of use, such as avoiding severe shaking of the preparation, passing the preparation through a filter at the time of dissolution or administration.

From such a background, it is an extremely important task to enhance stability of proteins to various kinds of stress, and there is a need for creation of a highly versatile stabilization technique which is applicable particularly to proteins in the state of high concentration and to proteins liable to form aggregates.

In view of the above, as a tackling for enhancing the stability of proteins, a large number of investigations have been made of stabilization of the proteins mainly by various additives. In Patent Document 1, a method of stabilizing proteins by addition of an amino acid ester or a polyamine is disclosed. Besides, in Patent Document 2, there is disclosed a method of stabilizing a protein against thermal stress by coexistence of the protein with polyethylene glycol and a specific amino acid such as alanine, arginine, glutamic acid or their derivatives.

On the other hand, in regard of complexes of a protein and a polyamino acid, investigations of the complexes as a drug delivery system have also been made. In Patent Document 3, it is disclosed that complexes of a hydrophobicized polyamino acid and an antigen protein or antigen polypeptide can be utilized as immunoadjuvant for enhancing immunogenicity, aiming at imparting stability and functionality in a living body. In addition, in Patent Document 4, complexes using a polyamino acid as one of cationic polymers is disclosed as a technology which reduces interactions between a contained protein and a polymer matrix constituting a delivery system and which is incorporated into a system exhibiting excellent sustained release properties. However, the document does not discuss stability of aqueous suspension preparations containing these complexes during transportation or during storage.

In Patent Document 5, "a method of making an antibody formulation comprising combining an antibody with a polycation" is described in claim 1. It is described in claim 4 that "the polycation is selected from the group consisting of polylysine, polyarginine, polyornithine, polyhistidine, and cationic polysaccharides, or mixtures thereof," it is described in claim 24 that "the formulation has a greater solubility when combined with the polycation composition as compared to the solubility of the antibody formulation in the absence of polycations," and it is described in claim 25 that "the formulation has an increased shelf-life as compared to such an antibody formulation that has not been combined with a polycation composition." However, there is no description about formation of solid particles, which are a complex of an antibody and a polyamino acid, or about combinations of an antibody and a polyanion. In Patent Document 5, it is described that "in one embodiment, the antibody has a greater solubility in water than a composition containing the antibody or Fc-fusion molecule in the absence of a polycation" (see [0021]).

### Prior Art Documents

### Non-patent Document

Non-patent Document 1: Schellekens, H. Clin. Therapeutics, 24(11): 1720-1740. 2002

### Patent Documents

Patent Document 1: Japanese Patent No. 3976257
Patent Document 2: Japanese Patent No. 5119545
Patent Document 3: PCT Patent Publication No. WO2010/110455
Patent Document 4: Japanese Translations of PCT for Patent No. 1995-503700
Patent Document 5: Japanese Translations of PCT for Patent No. 2010-536786

### Summary of Invention

### Technical Problem

An object of the present invention, which is made in consideration of the above-mentioned circumstances, is to provide an aqueous suspension preparation containing a protein, being excellent in stability during transportation and during storage and being excellent in handleability when put to use. The present inventors made earnest studies on a variety of proteins for the aforesaid object. As a result of the studies, the present inventors have found out that a protein and a polyamino acid form a complex which is suspended in a buffer, and, in the complex, the protein has at least one of shaking stress resistance, fluidity enhancement, oxidation resistance, thermal stability, and aggregation inhibitory properties, thereby being enhanced in stability, and they have come to make the present invention.

### Technical Solution

The present inventors have found out that a method including a step of combining a particular protein with an anionic polyamino acid causes the protein to have shaking stress resistance and be enhanced in stability, and they have reached the present invention.

Namely, the present invention provides the method set out in the appended set of claims.
In embodiments of the method according to the invention:
- the aqueous suspension preparation having the fluidity has the complex being low in viscosity as compared to an aqueous solution of the same protein at the same concentration in the same buffer;
- the aqueous suspension preparation having the oxidation resistance has the complex being high in oxidation resistance as compared to an aqueous solution of the same protein at the same concentration in the same buffer;
- the aqueous suspension preparation having the thermal resistance has the complex being high in thermal resistance as compared to an aqueous solution of the same protein at the same concentration in the same buffer;
- the aqueous suspension preparation having the aggregation inhibitory properties has the complex being high in aggregation inhibitory properties as compared to an aqueous solution of the same protein at the same concentration in the same buffer.
The invention also relates to a protein-containing aqueous liquid obtained by adding a low-concentration electrolyte to the aqueous suspension containing the complex as defined in the invention, to dissolve the protein.

### Advantageous Effects

In the method of stabilizing a protein of the present invention, a protein and a polyamino acid that have surface charge in a buffer form a complex to be suspended in the buffer, and the protein in the complex is not deteriorated when concentrated as it has at least one effect of shaking stress resistance, fluidity enhancement, oxidation resistance, thermal resistance, and aggregation inhibitory properties. Specifically, the protein and the polyamino acid form a complex, whereby the protein is stabilized and is enabled to be concentrated by removing water from the aqueous suspension. The obtained aqueous suspension preparation is excellent in stability during transportation and during storage, and is excellent in handleability when put to use. In general, for stabilization of protein drugs, use is made of additives such as excipients, surfactants, stabilizers, and when these additives are used, a lot of investigations are required for optimizing the kinds, combinations, and addition amounts of the additives on the basis of each of the protein preparations. The methods of the present invention stabilize a particular protein easily by forming a complex of the protein with an anionic polyamino acid, and eliminates the need for addition of additives that has conventionally been necessary. Besides, the aqueous suspension preparation obtained by the method of the present invention does not need a complicated dissolving operation, which is needed for freeze-dried pharmaceutical preparations, and can be administered as it is or administered in the form of an aqueous liquid obtained through addition of an inorganic salt represented by sodium chloride when put to use. Furthermore, the aqueous suspension preparation obtained by the method of the present invention is characterized by being low in viscosity even when it contains the protein in a high concentration. Therefore, the amount of the aqueous suspension preparation which would be left uselessly in a container when put to use can be reduced. Besides, when administered by use of a syringe, the aqueous suspension preparation can be administered with a weak force as compared to an aqueous solution containing the protein in the same concentration.

The aqueous suspension preparation obtained by the method of the present invention has one of the above-mentioned characteristic features.

### Brief Description of Drawings

[FIG. 1]

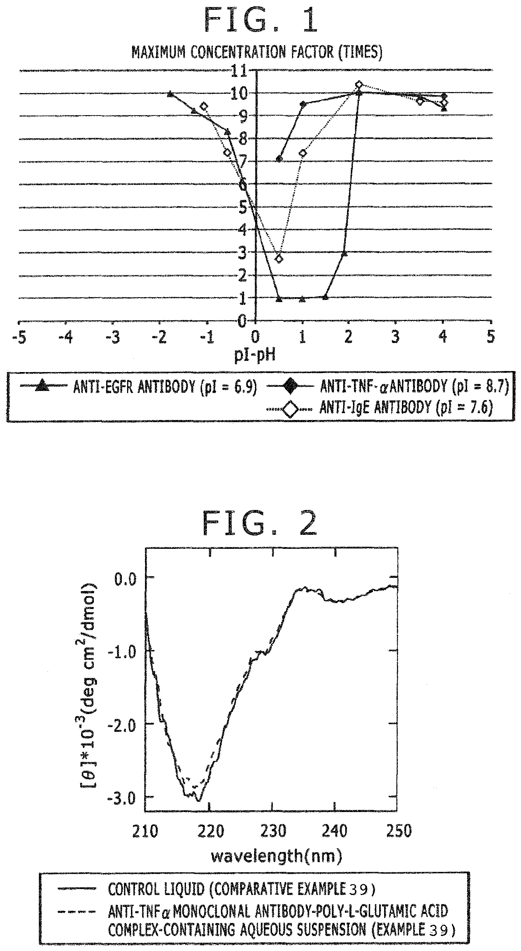

   FIG. 1 depicts graphs depicting that maximum concentration factors of complexes of protein and polyamino acid are present where the absolute value of the difference between pH of a buffer and isoelectric point pI of the protein is in the range from 0.5 to 4.0.
[FIG. 2]
   FIG. 2 depicts graphs obtained in Test Example 13 to be described later by measurement of CD spectrum for Example 39 (broken line) in which an anti-TNFα monoclonal antibody-poly-L-glutamic acid complex (in MOPS buffer) is formed, concentrated and re-dissolved, and for Comparative Example 39 (solid line) containing no polyamino acid and not being concentrated, as a control liquid. The CD spectra of both samples agree with each other, indicating that a secondary structure of the protein is maintained unchanged after the complex is formed and then solubilized.
[FIG. 3]

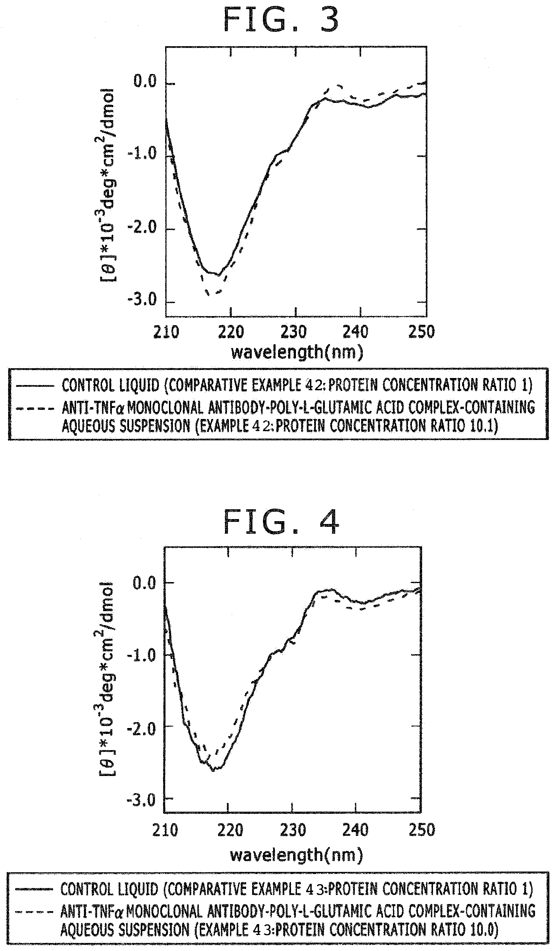

   FIG. 3 depicts graphs obtained in Test example 14 to be described later by measurement of CD spectrum for Example 42 (broken line) in which an anti-TNFα monoclonal antibody-poly-L-glutamic acid complex (in MOPS buffer) is formed, concentrated and then re-dissolved, and for Comparative Example 42 (solid line) containing no polyamino acid and not being concentrated, as a control liquid. The CD spectra of both samples agree with each other, indicating that a secondary structure of the protein is maintained unchanged after the complex is formed and then solubilized.
[FIG. 4]
   FIG. 4 depicts graphs obtained in Test Example 15 to be described later by measurement of CD spectrum for Example 43 (broken line) in which an anti-TNFα monoclonal antibody-poly-L-glutamic acid complex (in citrate buffer) is formed, concentrated and then re-dissolved, and for Comparative Example 43 (solid line) containing no polyamino acid and not being concentrated, as a control liquid. The CD spectra of both samples agree with each other, indicating that a secondary structure of the concentrated protein is maintained unchanged when the complex is formed and then solubilized.
[FIG. 5]

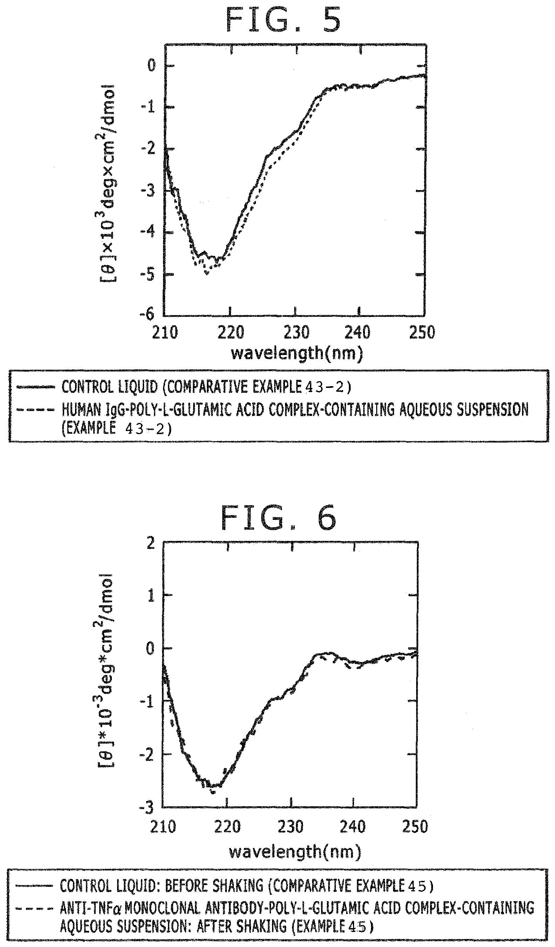

   FIG. 5 depicts graphs obtained in Test Example 15-2 to be described later by measurement of CD spectrum for Example 43-2 (broken line) in which a human IgG-poly-L-glutamic acid complex (in citrate buffer) is formed, concentrated and then re-dissolved, and for Comparative Example 43-2 (solid line) containing no polyamino acid and not being concentrated, as a control liquid. The CD spectra of both samples agree with each other, indicating that a secondary structure of the concentrated protein is maintained unchanged when the complex is formed and then solubilized.
[FIG. 6]
   FIG. 6 depicts graphs depicting CD spectra measured in Test Example 26 to be described later, after shaking (broken line) of an anti-TNFα monoclonal antibody-poly-L-glutamic acid complex-containing aqueous suspension (Example 45), and before shaking (solid line) of a control liquid (Comparative Example 45) containing no polyamino acid. The CD spectrum obtained for the case in which shaking stress is applied to the anti-TNFα monoclonal antibody-poly-L-glutamic acid complex-containing aqueous suspension and for the case in which shaking stress is not applied agree with each other, indicating that a secondary structure of the protein is maintained even when shaking stress is applied to the protein in the complex.
[FIG. 7]

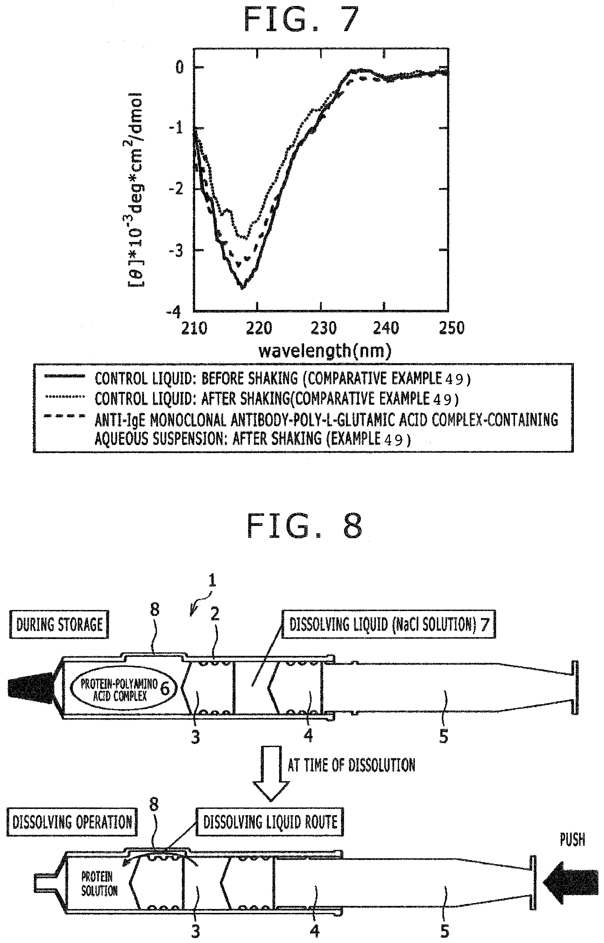

   FIG. 7 depicts graphs depicting CD spectra measured in Test Example 27 to be described later, after shaking (broken line) of an anti-IgE monoclonal antibody-poly-L-glutamic acid complex-containing aqueous suspension (Example 49), and before shaking (solid line) and after shaking (broken line) of a control liquid (Comparative Example 49) containing no polyamino acid. The CD spectrum for the case where shaking stress is applied to the control liquid and the CD spectrum for the case where shaking stress is not applied do not agree with each other, indicating that a change in secondary structure is generated. On the other hand, the CD spectrum for the case where shaking stress is applied to the anti-IgE monoclonal antibody-poly-L-glutamic acid complex-containing aqueous suspension and the CD spectrum for the case where shaking stress is not applied agree with each other, indicating that the secondary structure of the protein is maintained unchanged even when shaking stress is applied to the protein in the complex.
[FIG. 8]
   FIG. 8 is a schematic view illustrating operations of a two-gas-chamber prefilled syringe during storage and at the time of dissolution.
[FIG. 9]

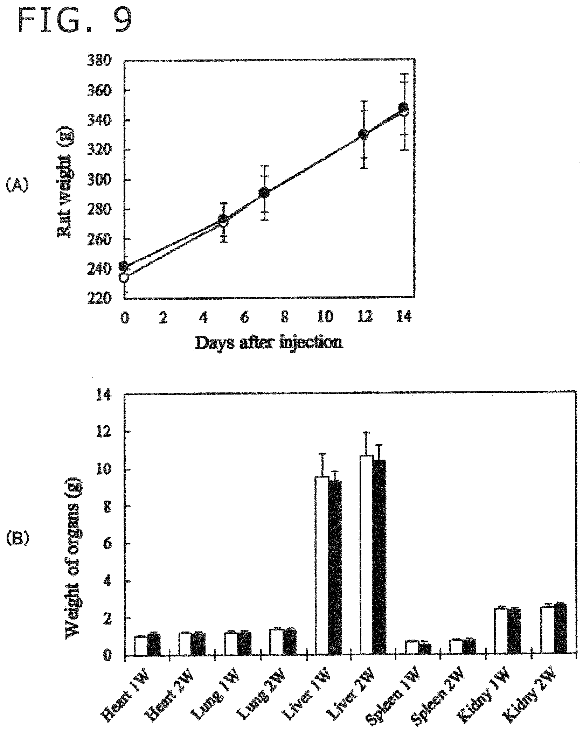

   FIG. 9(A) is a graph depicting variations in body weight of rats subjected to subcutaneous injection of a rat IgG citrate buffer (pH 5.0) solution (white circles) and a rat IgG-polyglutamic acid complex-containing aqueous suspension (black circles). The axis of ordinates represents rat's body weight (g) and the axis of abscissas represents the number of days after injection. There was observed no significant difference in rat's body weight between the rat IgG citrate buffer (pH 5.0) solution administration group (white circles) and the rat IgG-polyglutamic acid complex-containing aqueous suspension administration group (black circles). FIG. 9(B) is a graph obtained by measurement of weights of organs after one week and after two weeks from subcutaneous injection of a rat IgG citrate buffer (pH 5.0) solution and a rat IgG-polyglutamic acid complex-containing aqueous suspension into rats. There was observed no significant difference in any rat organ weight between the rat IgG citrate buffer (pH 5.0) solution administration group (white bars) and the rat IgG-polyglutamic acid complex-containing aqueous suspension administration group (black bars).
[FIG. 10]

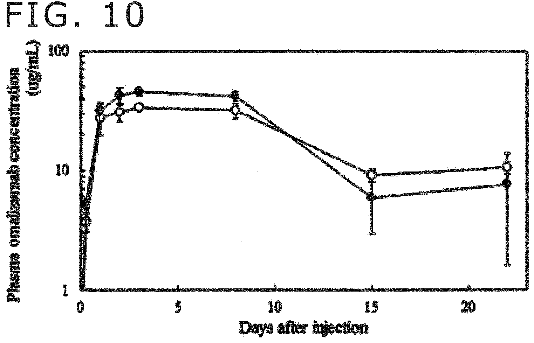

   FIG. 10 is a graph depicting transition of concentration of anti-IgE monoclonal antibody in plasma after subcutaneous injection of an anti-IgE monoclonal antibody solution (white circles) and an anti-IgE monoclonal antibody-polyglutamic acid complex-containing aqueous suspension (black circles) into rats. The axis of ordinates represents the concentration of anti-IgE monoclonal antibody in rat plasma, and the axis of abscissas represents the number of days after injection. The plasma anti-IgE monoclonal antibody concentration-time curve for the anti-IgE monoclonal antibody solution administration group (white circles) and that for the anti-IgE monoclonal antibody-polyglutamic acid complex-containing aqueous suspension administration group (black circles) were approximate to each other.

### Modes for Carrying Out the Invention

The present invention will be described specifically below by depicting examples of the protein-polyamino acid complex-containing aqueous suspension preparation obtained by the method of the present invention, but the present invention is not limited to these examples.

### [Protein]

A protein for use in the present invention is not specifically restricted so long as it is a protein which is of high purity and has a surface charge in a buffer. The protein is at least one selected from group consisting of anti-IgE monoclonal antibody, anti-TNFα monoclonal antibody, anti-TNFα monoclonal antibody Fab fragment, and anti-EGFR monoclonal antibody. In the present invention, proteins in more preferable form are proteins for medical use.

If the aqueous suspension preparation obtained by the method of the present invention is applied to a human as a pharmaceutical preparation or a diagnostic agent, the protein can be transported and stored more stably, and can be easily administered to the human when put to use, ensuring high usefulness.

The high purity in the case of proteins for medical use means a drug level, or a level such as to be usable as a drug for humans. In the cases of other proteins, the high purity means a high purity of reagent grade; for example, a threshold of not more than 0.1% by weight in total amount of impurities may be mentioned.

### [Polyamino acid]

A polyamino acid or its water-soluble salt for use in the present invention has a surface charge in a buffer. For the polyamino acid to be used in the complex or protein aqueous suspension preparation of the present invention to be described later, a high purity of reagent grade, for example, a threshold of not more than 0.15% by weight in total amount of impurities may be mentioned.

The polyamino acid is an anionic polyamino acid or a salt thereof. It is at least one selected from group consisting of polyglutamic acid, polyaspartic acid and their water-soluble salts. As examples thereof, there may be mentioned sodium salts of polyglutamic acid and polyaspartic acid. As for the form of the polyamino acid of more preferable form, there may be mentioned, as examples of the polyamino acid, poly-L-glutamic acid sodium salt, and poly-L-aspartic acid sodium salt. A 50% cell growth inhibitory concentration of the polyamino acid is preferably not less than 0.2%, more preferably not less than 1.0%.

### [Buffer]

A buffer for use in the present invention refers to a solution having a buffering action on hydrogen ion concentration. The buffer is an aqueous solution so controlled that its pH is not largely varied even when a small amount of acid or base is added to the solution or when its concentration is somewhat changed. The buffer is not particularly limited so long as it is ordinarily in chemical use, but preferably is a buffer for biological use. As a buffer of more preferable form, there can be used buffers which are suitable for biosamples and which can be administered into living bodies, such as phosphate buffer, citrate buffer, MOPS buffer (3-(N-morpholino)propanesulfonic acid) buffer, PIPES (Piperazine-1,4-bis(2-ethanesulfonic acid)) buffer, Tris-HCl buffer, MES (2-Morpholinoethanesulfonic acid, monohydrate) buffer, HEPES (4-(2-Hydroxyethyl)-1-piperazine ethanesulfonic acid) buffer, glycine NaOH buffer.

### [Protein-Polyamino Acid Complex]

### 1. Protein-Polyamino Acid Complex

The complex in the present invention has the following characteristic properties (1) to (4).
(1) The complex of a protein and a polyamino acid in the present invention is obtained in a complex-containing aqueous suspension in which the protein and the polyamino acid form the complex in a buffer to be suspended in the buffer.
(2) The protein can be concentrated by forming the complex in the buffer and removing at least part of water from the complex-containing aqueous suspension obtained.
(3) The complex can be dissolved by adding a low-concentration electrolyte to the complex-containing aqueous suspension.
(4) The protein is stable in the complex, and its activity is not deteriorated when it is concentrated.

The complex in the present invention is a complex formed when a protein and a polyamino acid that have surface charge are contained in a buffer and the protein and the polyamino acid interact with each other, preferably on an electrostatic basis. For the complex, a concentration factor in excess of 1 can be obtained when the absolute value of the difference between pH of the buffer and isoelectric point pI of the protein is in the range of preferably from 0.5 to 4.0. As depicted in FIG. 1, independently of the kind of the protein, the concentration factor increases where |pH - pI| ranges from about 0.5 to 2, a concentration factor of about 10 times being obtained, and thereafter a concentration factor remaining on the same level is exhibited irrespectively of the value of |pH - pI|. In other words, it is seen that suitable concentration can be achieved when |pH - pI| is in the range of preferably 0.5 to 4.0, more preferably from 1.0 to 4.0, more preferably from 1.0 to 3.5, more preferably from 1.8 to 3.5, more preferably from 1.8 to 3.0, and further preferably from 1.8 to 2.5.

It is not elucidated why the maximum concentration factor of the protein in the complex of the protein and the polyamino acid lies where the absolute value of the difference between the pH of the buffer and the isoelectric point pI of the protein is in the range from 0.5 to 4.0. The present inventors consider that the fact that the maximum concentration factor of the protein in the complex is present where the absolute value of the difference between the pH of the buffer and the isoelectric point pI of the protein is in the range of preferably from 1.5 to 4.0, more preferably from 1.0 to 4.0, more preferably from 1.0 to 3.5, more preferably from 1.5 to 3.5, more preferably from 1.8 to 3.5, more preferably from 1.8 to 3.0, and further preferably from 1.8 to 2.5 is one characteristic that represents a state of an electrostatic interaction by which the protein and the polyamino acid form the complex, but the present invention is not limited to these mechanisms.

The protein is stable in the complex, and its activity is not deteriorated when it is concentrated.

Here, that the activity is not deteriorated refers to that even when the complex is subjected to a concentrating operation, its biological activity is retained at a level of not less than 80% based on that in a control liquid of the same protein at the same concentration in the same buffer. More preferably, the level is not less than 85%, or not less than 90%. In some cases, the measured value exceeds 100%, and it is not clear whether such a measured value is due to measurement errors or due to some mechanism.

In addition, the protein is stable in the complex, the secondary structure of the protein is retained after the complex is formed and then solubilized, and the secondary structure of the protein is retained even after concentration. It is known that even when a protein forms the complex in the present invention and subjected to concentration and then to re-dissolution, the secondary structure of the protein is little changed. This has been verified by measurement of the CD spectra before formation of the complexes and the CD spectra after the complex formation and re-dissolution, as depicted in FIGS. 2, 3, 4, and 5 which respectively depict the results of Examples 39, 42, 43, and 43-2 to be described later. While the concentration in the concentrated state of the high-purity protein used in the present invention is not limited, it is estimated from the aforesaid experiments that the secondary structure of the protein is not changed when the protein is used at a concentration of preferably from 0.1 mg/mL to 300 mg/mL.

The protein-polyamino acid complexes in the present invention can be described as complexes [1] to [5] which are characterized by the following five characteristic properties and have different uses according to their characteristic properties.

### [1. Protein-polyamino acid complex having shaking stress resistance (hereinafter referred to as complex [1])]

The complex [1] in the present invention has the following characteristic properties (1) to (5).
(1) The complex [1] of a protein and a polyamino acid in the present invention is obtained in a complex-containing aqueous suspension in which the protein and the polyamino acid form the complex in a buffer to be suspended in the buffer.
(2) The protein can be concentrated by forming the complex [1] in the buffer and removing at least part of water from the complex-containing aqueous suspension obtained.
(3) The complex [1] can be dissolved by adding a low-concentration electrolyte to the complex-containing aqueous suspension.
(4) The activity of the protein is not damaged when the protein is concentrated using the complex in the present invention. Besides, the secondary structure of the protein is retained after the protein is subjected to complex formation and then solubilization, and the protein retains its secondary structure even when concentrated.
(5) The protein has shaking stress resistance in the complex [1].

The characteristic properties (1) to (4) are the same as described in 1. Protein-Polyamino Acid Complex above, and, therefore, the descriptions thereof are omitted here. The characteristic property (5) will be described below.

### (5) The protein has shaking stress resistance in the complex.

Here, to have shaking stress resistance refers to that the complex [1] has a high shaking stress resistance, specifically, has a high activity retention rate, or retains the structure of the protein better, or has a high protein retention rate, as compared with a control liquid of the same protein at the same concentration in the same buffer.

The shaking stress refers to application of shaking of, for example, 100 rpm to 500 rpm for 10 hours to 100 hours, as indicated in Test Examples in Examples described later, to a protein or complex in a buffer. The shaking stress resistance, which differs according to the kind of protein and hence cannot be restricted, refers to that activity retention rate, or the ratio of protein activity after shaking stress to protein activity before shaking stress, is 10% to 99% higher than that in the case where not any complex is formed. Preferably, the activity retention rate may be 10% to 50% higher, more preferably 15% to 50% higher.

In addition, when the concentration of a protein is measured in terms of absorbance before and after the shaking stress, the following situations are observed. In the case where not any complex is formed, a large amount of insoluble precipitate is formed, so that when aggregates are removed by centrifugation after shaking, the concentration of the protein in the buffer is lowered, and the retention rate of the protein content is lowered, down to below 7% in some cases. On the other hand, in the case where the complex is formed, the protein in the complex is stable before and after the shaking stress, and the retention rate of the protein content may be 100% or higher depending on measurement. The value of retention rate of the content of the protein in the complex is preferably 15% to 99% higher, more preferably 18% to 95% higher, and further preferably 25% to 90% higher, than the value of retention rate of the content of the protein in the control liquid in which not any complex is formed.

### [2. Protein-polyamino acid complex having fluidity (hereinafter referred to as complex [2])]

The complex [2] in the present invention has the following characteristic properties (1) to (5).
(1) The complex [2] of a protein and a polyamino acid in the present invention is obtained in a complex-containing aqueous suspension in which the protein and the polyamino acid form the complex in a buffer to be suspended in the buffer.
(2) The protein can be concentrated by forming the complex [2] in the buffer and removing at least part of water from the complex-containing aqueous suspension obtained.
(3) The complex [2] can be dissolved by adding a low-concentration electrolyte to the complex-containing aqueous suspension.
(4) The activity of the protein is not damaged when the protein is concentrated using the complex [2] in the present invention. Besides, the secondary structure of the protein is retained after the protein is subjected to complex formation and then solubilization, and the protein retains its secondary structure even when concentrated.
(5) The protein is excellent in fluidity in the complex [2].

The characteristic properties (1) to (4) are the same as described in 1. Protein-Polyamino Acid Complex above, and, therefore, the descriptions thereof are omitted here. The characteristic property (5) will be described below.

### (5) The protein is excellent in fluidity in the complex [2].

Here, to have fluidity, to be excellent in fluidity, or fluidity enhancement refers to that the complex [2] is more fluid than a control liquid of the same protein at the same concentration in the same buffer; specifically, it refers to that the viscosity of the protein contained in the complex [2] is low, as compared at the same concentration in the same buffer.

The viscosity of the protein in the complex [2] differs depending on the kind and concentration of the protein, and is not limited. The viscosity may be not more than 60%, preferably not more than 55%, and further preferably not more than 50%, based on the viscosity of the control liquid.

### [3. Protein-polyamino acid complex having oxidation resistance (hereinafter referred to as complex [3])]

The complex [3] in the present invention has the following characteristic properties (1) to (5).
(1) The complex [3] of a protein and a polyamino acid in the present invention is obtained in a complex-containing aqueous suspension in which the protein and the polyamino acid form the complex in a buffer to be suspended in the buffer.
(2) The protein can be concentrated by forming the complex [3] in the buffer and removing at least part of water from the complex-containing aqueous suspension obtained.
(3) The complex [3] can be dissolved by adding a low-concentration electrolyte to the complex-containing aqueous suspension.
(4) The activity of the protein is not damaged when the protein is concentrated using the complex [3] in the present invention. Besides, the secondary structure of the protein is retained after the protein is subjected to complex formation and then solubilization, and the protein retains its secondary structure even when concentrated.
(5) The protein has oxidation resistance in the complex [3].

The characteristic properties (1) to (4) are the same as described in 1. Protein-Polyamino Acid Complex above, and, therefore, the descriptions thereof are omitted here. The characteristic property (5) will be described below.

### (5) The protein has oxidation resistance in the complex [3].

Here, oxidation stress refers to a change generated in the primary structure of a protein as a result of a process in which an active oxygen species or the like in an oxidizing compound added brings about fragmentation, association, modification or the like in an amino acid side chain or chains susceptible to oxidation in the protein. To have oxidation resistance refers to that the complex [3] is higher in oxidation resistance than a control liquid of the same protein at the same concentration in the same buffer, specifically that the activity retention rate of the protein in the complex [3] in response to addition of an oxidizing compound is high, or the structure of the protein is retained, as compared to a control liquid to which the same compound is added, at the same concentration in the same buffer.

As an example of the oxidizing compound to be used, there can be mentioned a mixture of 0.01% by weight to 3.00% by weight aqueous hydrogen peroxide and a buffer, 1 mM to 10 mM ascorbic acid in the presence of 1 mM to 10 mM of copper ions, and the like. A change in the activity of the protein or a change in the primary structure of the protein is measured, after the system is kept for a predetermined time at a predetermined temperature. The predetermined time and the predetermined temperature may be, for example, one hour to 10 hours and 20°C to 40°C.

The oxidation resistance of the protein in the complex [3] differs depending on the kind and concentration of the protein and is therefore not restricted. As for the difference from the retained activity of a control liquid under the same oxidation stress, the retained activity of the protein in the complex [3] may be 5% to 30% higher, preferably 5% to 25% higher, and further preferably 10% to 20% higher.

Besides, as for retention of the structure of the protein, let the change rate of primary structure in a control liquid (Comparative Example) in which not any complex is formed, under the same oxidation stress, be 100%, then the change rate of primary structure of the protein in the complex is suppressed to preferably 95% or below, more preferably 91% or below, and further preferably 87% or below.

### [4. Protein-polyamino acid complex having thermal resistance (hereinafter referred to as complex [4])]

The complex [4] in the present invention has the following characteristic properties (1) to (5).
(1) The complex [4] of a protein and a polyamino acid in the present invention is obtained in a complex-containing aqueous suspension in which the protein and the polyamino acid form the complex in a buffer to be suspended in the buffer.
(2) The protein can be concentrated by forming the complex [4] in the buffer and removing at least part of water from the complex-containing aqueous suspension obtained.
(3) The complex [4] can be dissolved by adding a low-concentration electrolyte to the complex-containing aqueous suspension.
(4) The activity of the protein is not damaged when the protein is concentrated using the complex [4] in the present invention. Besides, the secondary structure of the protein is retained after the protein is subjected to complex formation and then solubilization, and the protein retains its secondary structure even when concentrated.

### (5) The protein has thermal resistance in the complex [4].

The characteristic properties (1) to (4) are the same as described in 1. Protein-Polyamino Acid Complex above, and, therefore, the descriptions thereof are omitted here. The characteristic property (5) will be described below.

Here, to have thermal resistance refers to that the complex [4] is higher in thermal resistance than a control liquid of the same protein at the same concentration in the same buffer, specifically that the activity of the protein in the complex [4] after thermal load is high, or the denaturing temperature of the protein is high, as compared to a control liquid in which not any complex is formed, at the same concentration in the same buffer.

The thermal load refers to application of heat to the complex [4] or to a control liquid of the same protein at the same concentration in the same buffer, at a predetermined temperature for a predetermined time, for example, at room temperature to 60°C for five minutes to 20 hours. The thermal resistance can be evaluated by a method in which the activity in the case where the complex [4] or a control liquid of the same protein at the same concentration in the same buffer is stored in a cold place is assumed to be 100%, and the activity retention rate in the complex [4] after the aforesaid thermal load or the control liquid of the same protein at the same concentration in the same buffer is measured. Alternatively, the thermal resistance can be evaluated by a method in which the complex [4] and the control liquid of the same protein at the same concentration in the same buffer are put to differential scanning calorimetry to measure the respective denaturing temperatures.

The thermal resistance of the protein in the complex [4] differs depending on the kind and concentration of the protein, and is not restricted. The difference from the activity detention rate in the control liquid under the same thermal stress may be 5% to 95%, preferably 10% to not more than 95%, and further preferably 20% to 85%.

The thermal denaturing temperature of the protein in the complex [4] is preferably 1°C to 20°C high, as compared to the control liquid.

### [5. Protein-polyamino acid complex having aggregation inhibitory properties (hereinafter referred to as complex [5])]

The complex [5] in the present invention has the following characteristic properties (1) to (5).
(1) The complex [5] of a protein and a polyamino acid in the present invention is obtained in a complex-containing aqueous suspension in which the protein and the polyamino acid form the complex in a buffer to be suspended in the buffer.
(2) The protein can be concentrated by forming the complex [5] in the buffer and removing at least part of water from the complex-containing aqueous suspension obtained.
(3) The complex [5] can be dissolved by adding a low-concentration electrolyte to the complex-containing aqueous suspension.
(4) The activity of the protein is not damaged when the protein is concentrated using the complex [5] in the present invention. Besides, the secondary structure of the protein is retained after the protein is subjected to complex formation and then solubilization, and the protein retains its secondary structure even when concentrated.
(5) The protein has aggregation inhibitory properties in the complex [5].

The characteristic properties (1) to (4) are the same as described in 1. Protein-Polyamino Acid Complex above, and, therefore, the descriptions thereof are omitted here. The characteristic property (5) will be described below.

Here, to have aggregation inhibitory properties refers to that the complex [5] is superior in aggregation inhibition to a control liquid of the same protein at the same concentration in the same buffer, and specifically that the formation of aggregates is restrained by the formation of the complex. The formation of aggregates can be evaluated by a method in which aggregation amounts of protein before and after shaking stress in the complex [5] and in a control liquid of the same protein at the same concentration in the same buffer are calculated by subtracting the mass of protein in the supernatant from the total mass of protein, or a method in which soluble aggregate peak area according to size exclusion chromatography is measured. Alternatively, the formation of aggregates can be evaluated by calculating the increase rate of the number of aggregates of protein or the increase rate of turbidity, before and after thermal stress, for the complex [5] and a control liquid of the same protein at the same concentration in the same buffer. By these measurements, it can be evaluated that the protein in the complex [5] is restrained from the formation of aggregates under various conditions.

The aggregation inhibitory properties of the protein in the complex [5] differ depending on the kind and concentration of the protein, and are not restricted. The complex [5] may have such a characteristic property that the difference in insoluble aggregate formation rate from the control liquid under the same shaking stress is 5% to 20%, preferably that aggregates are substantially absent after the shaking stress. The increase rate of soluble aggregates under the same shaking stress, in terms of ratio to that of the control liquid, may be 30% or below, preferably 10% or below, and further preferably 5% or below. The increase rate of the number of aggregates under the same shaking stress, in terms of ratio to that of the control liquid, may be 1.0% or below, preferably 0.5% or below, and further preferably 0.2% or below. The increase rate of turbidity under the same thermal stress, in terms of ratio to that of the control liquid, may be 10% or below, preferably 6% or below, and further preferably 4% or below.

### [Characteristic properties of aqueous suspension preparation containing protein-polyamino acid complex]

The protein has a positive or negative surface charge. The polyamino acid is anionic.

The molecular weight of the anionic polyamino acid is preferably in the range from 0.5 kDa to 1000 kDa. More preferably, the molecular weight is 5 kDa to 800 kDa, and further preferably 10 kDa to 500 kDa. The molecular weight of the protein is preferably in the range from 3 kDa to 670 kDa. The 50% cell growth inhibitory concentration of the polyamino acid is preferably not less than 0.2%, more preferably not less than 1.0%.

The anionic polyamino acid is preferably combined with a protein which has a positive surface charge.

The protein is contained in the aqueous suspension at a concentration in the range from 0.01 mg/mL to 500 mg/mL.

The pH of the buffer used for the aqueous suspension is preferably in the range from pH 3 to pH 10.5.

The aqueous suspension preparation obtained by the method of the present invention is a stable protein-polyamino acid complex-containing aqueous suspension preparation which contains a protein and a polyamino acid that have surface charge in a buffer, wherein the blending ratio of these ingredients is preferably such as to contain 0.005 parts by mass to 6 parts by mass of the polyamino acid based on 1 part by mass of the protein, the pH of the buffer is adjusted to be deviated from the isoelectric point pI of the protein contained therein to the basic side or acidic side by not less than 0.5, and the protein and the polyamino acid form a complex by electrostatic interaction.

The aqueous suspension preparation of the present invention can be concentrated and stabilized easily by only selecting the pH of the buffer and the polyamino acid according to the isoelectric point of the protein, and does not need addition of an additive or additives, which has been necessary conventionally. An additive or additives other than the buffer, protein, polyamino acid, and pH adjuster can be added to the aqueous suspension preparation, but it is unnecessary to add such additives. Besides, the aqueous suspension preparation does not need an intricate dissolving operation required for a freeze-dried pharmaceutical preparation, and can be administered as it is as a pharmaceutical preparation. Alternatively, the aqueous suspension preparation can be administered as a pharmaceutical preparation after adding an inorganic salt represented by sodium chloride thereto and dissolving it.

### [Combination of protein and polyamino acid for forming protein-polyamino acid complex]

The complex in the present invention contains a protein and an anionic polyamino acid.
1. (1) In the case of a protein (antibody fragment as defined above) having an isoelectric point in an acidic region (pH not less than 3.0 and less than 6.0), in the case of using a buffer having a buffer capacity on the acidic side of the isoelectric point, at least one anionic polyamino acid selected from the group consisting of polyglutamic acid (MW: 750 to 5000, pI: 2.81 to 3.46), polyglutamic acid (MW: 3000 to 15000, pI: 2.36 to 3.00), polyglutamic acid (MW: 15000 to 50000, pI: 1.85 to 2.36), polyglutamic acid (MW: 50000 to 100000, pI: 1.56 to 1.85), polyaspartic acid (MW: 2000 to 11000, pI: 2.06 to 2.75), polyaspartic acid (MW: 5000 to 15000, pI: 1.93 to 2.39) and their water-soluble salts is added, to form a complex.
   (2) As buffer for the protein having the isoelectric point in an acidic region, known buffers can be used appropriately, without any particular restrictions. As buffer, there may be used, for example, phosphate buffer, citrate buffer, citrate-phosphate buffer, tartarate buffer, trishydroxymethylaminomethane-HCl buffer (tris-hydrochloric acid buffer), MES buffer (2-morpholinoethanesulfonic acid buffer), TES buffer (N-tris(hydroxymethyl)methyl-2-aminoethanesulfonic acid buffer), acetate buffer, MOPS buffer (3-morpholinopropanesulfonic acid buffer), MOPS-NaOH buffer, HEPES buffer (4-(2-hydroxyethyl)-1-piperazineethanesulfonic acid buffer), HEPES-NaOH buffer, PIPES buffer (piperazine-1,4-bis(2-ethanesulfonic acid) buffer), and the like GOOD buffers, glycine-hydrochloric acid buffer, glycine-NaOH buffer, glycylglycine-NaOH buffer, glycylglycine-KOH buffer and the like amino acid buffers, Tris-boric acid buffer, boric acid-NaOH buffer, borate buffer and the like boric acid buffers, or imidazole buffer.
2. (1) In the case of a protein (antibody or antibody fragment as defined above) having an isoelectric point in a neutral region (pH 6.0 to 8.0), in the case of using a buffer having a buffer capacity on the acidic side of the isoelectric point, at least one anionic polyamino acid selected from the group consisting of polyglutamic acid (MW: 750 to 5000, pI: 2.81 to 3.46), polyglutamic acid (MW: 3000 to 15000, pI: 2.36 to 3.00), polyglutamic acid (MW: 15000 to 50000, pI: 1.85 to 2.36), polyglutamic acid (MW: 50000 to 100000, pI: 1.56 to 1.85), polyaspartic acid (MW: 2000 to 11000, pI: 2.06 to 2.75), polyaspartic acid (MW: 5000 to 15000, pI: 1.93 to 2.39) and their water-soluble salts is added, to form a complex.
   (2) As buffer for the protein having the isoelectric point in a neutral region, known buffers can be used appropriately, without any particular restrictions. As buffer, there may be used, for example, phosphate buffer, citrate buffer, citrate-phosphate buffer, tartarate buffer, trishydroxymethylaminomethane-HCl buffer (Tris-hydrochloric acid buffer), MES buffer (2-morpholinoethanesulfonic acid buffer), TES buffer (N-tris(hydroxymethyl)methyl-2-aminoethanesulfonic acid buffer), acetate buffer, MOPS buffer (3-morpholinopropanesulfonic acid buffer), MOPS-NaOH buffer, HEPES buffer (4-(2-hydroxyethyl)-1-piperazineethanesulfonic acid buffer), HEPES-NaOH buffer, PIPES buffer (piperazine-1,4-bis(2-ethanesulfonic acid) buffer), and the like GOOD buffers, glycine-hydrochloric acid buffer, glycine-NaOH buffer, glycylglycine-NaOH buffer, glycylglycine-KOH buffer and the like amino acid buffers, Tris-boric acid buffer, boric acid-NaOH buffer, borate buffer and the like boric acid buffers, or imidazole buffer.
3. (1) In the case of a protein (antibody or antibody fragment as defined above) having an isoelectric point in a basic region (pH more than 8.0 and not more than 11.0), in the case of using a buffer having buffer capacity on the acidic side of the isoelectric point, at least one anionic polyamino acid selected from the group consisting of polyglutamic acid (MW: 750 to 5000, pI: 2.81 to 3.46), polyglutamic acid (MW: 3000 to 15000, pI: 2.36 to 3.00), polyglutamic acid (MW: 15000 to 50000, pI: 1.85 to 2.36), polyglutamic acid (MW: 50000 to 100000, pI: 1.56 to 1.85), polyaspartic acid (MW: 2000 to 11000, pI: 2.06 to 2.75), polyaspartic acid (MW: 5000 to 15000, pI: 1.93 to 2.39) and their water-soluble salts is added, to form a complex.
   (2) As buffer for the protein having the isoelectric point in a basic region, known buffers can be used appropriately, without any particular restrictions. As buffer, there may be used, for example, phosphate buffer, citrate buffer, citrate-phosphate buffer, tartarate buffer, trishydroxymethylaminomethane-HCl buffer (Tris-hydrochloric acid buffer), MES buffer (2-morpholinoethanesulfonic acid buffer), TES buffer (N-tris(hydroxymethyl)methyl-2-aminoethanesulfonic acid buffer), acetate buffer, MOPS buffer (3-morpholinopropanesulfonic acid buffer), MOPS-NaOH buffer, HEPES buffer (4-(2-hydroxyethyl)-1-piperazineethanesulfonic acid buffer), HEPES-NaOH buffer, PIPES buffer (piperazine-1,4-bis(2-ethanesulfonic acid) buffer), and the like GOOD buffers, glycine-hydrochloric acid buffer, glycine-NaOH buffer, glycylglycine-NaOH buffer, glycylglycine-KOH buffer and the like amino acid buffers, Tris-boric acid buffer, boric acid-NaOH buffer, borate buffer and the like boric acid buffers, or imidazole buffer.

### [Method of preparing aqueous suspension preparation containing protein-polyamino acid complex]

(Step 1): The pH of a buffer is adjusted so that the pH is deviated to the basic side and/or the acidic side from the isoelectric point pI of the protein by an absolute value of 0.5 to 4.0.
(Step 2): To the buffer, the protein having a surface charge is added so as to attain a concentration in the range from 0.01 mg/mL to 50 mg/mL, and the polyamino acid is added so as to attain a concentration in the range from 0.01 mg/mL to 100 mg/mL, thereby forming an aqueous suspension which contains the protein-polyamino acid complex.
(Step 3): At least part of water or buffer is removed from the aqueous suspension containing the protein-polyamino acid complex by a method selected from among centrifugation, ultrafiltration, supernatant removal and the like, thereby making an adjustment such that the protein is contained at a concentration in the range from 0.1 mg/mL to 500 mg/mL.
(Step 4): To the aqueous suspension prepared in the step 3, a buffer or water or the like in an amount smaller than the amount of the water or buffer removed in the step 3 is added, to thereby obtain an aqueous suspension containing the protein-polyamino acid complex having a concentrated protein concentration. Alternatively, a buffer or water or the like in an amount equal to the amount of the water or buffer removed in the step 3 is added, to thereby obtain an aqueous suspension containing the protein-polyamino acid complex having an equal protein concentration. Yet alternatively, a buffer or water or the like in an amount larger than the amount of the water or buffer removed in the step 3 is added, thereby to obtain an aqueous suspension containing the protein-polyamino acid complex having a diluted protein concentration.
(Step 5): Without removing water from the complex-containing aqueous suspension obtained after the above step 1 and step 2, a buffer or water or the like is added to the aqueous suspension, to thereby obtain an aqueous suspension containing the protein-polyamino acid complex having a diluted protein concentration.

In the case where the protein is a protein for medical use, the aqueous suspension preparation is produced by the step 1, step 2, step 3, and step 4, or by the step 1, step 2, and step 5.

The pharmaceutical preparation of the present invention is an aqueous suspension protein preparation obtained by the method, in which a protein preparation and a polyamino acid that have surface charge in a buffer form a complex to be suspended in the buffer, wherein the protein preparation can be concentrated by removing at least part of water from the complex-containing aqueous suspension preparation, and the protein preparation is stabilized in the complex. The present invention is a method of stabilizing a medical protein by causing a protein preparation and a polyamino acid that have surface charge in a buffer to form a complex in the buffer, thereby obtaining an aqueous suspension preparation. The concentration of the complex in the aqueous suspension preparation may be enhanced, or the complex may be caused to gather to form a layer separated from a small amount or very small amount of water layer, resulting in a two-layer state. Besides, the complex may be separated as in a wet state. Further, a low-concentration electrolyte as an inorganic salt may be added to the complex in the aqueous suspension preparation, to allow dissolution, thereby obtaining an aqueous preparation and putting it to medical use. In the case where the pH upon dissociation of the aqueous suspension preparation by use of a salt is highly basic, for example pH 8 or above, or highly acidic, for example pH 4 or below, which is unsuitable for administration into a human, an acidic buffer or a basic buffer may be added, whereby the pH can be adjusted to the vicinity of a neutral point suitable for administration.

The protein preparation for medical use is not restricted, and is at least one of enzyme, cytokine, hormone, antibody, antibody fragment, and fusion protein for medical use.

Specific examples of the protein preparation for medical use include the following.

### (Antibodies)

muromonab-CD3, trastuzumab, rituximab, palivizumab, infliximab, basiliximab, tocilizumab, gemtuzumab ozogamicin, bevacizumab, ibritumomab tiuxetan, adalimumab, cetuximab, ranibizumab, omalizumab, eculizumab, panitumumab, ustekinumab, golimumab, canakinumab, denosumab, mogamulizumab, certolizumab prgol, ofatumumab, pertuzumab, trastuzumab emtansine, brentuximab vedotin, natalizumab, nivolumab, alemtuzumab, iodine 131-modified tositumomab, catumaxomab, adecatumumab, edrecolomab, abciximab, siltuximab, daclizumab, efalizumab, obinutuzumab, vedolizumab, pembrolizumab, ixekizumab, diridavumab, ipilimumab, belimumab, raxibacumab, ramucirumab.

The aqueous suspension preparation or aqueous preparation obtained by the method of the present invention does not have general toxicity, as will be depicted later. The aqueous suspension preparation or aqueous preparation is turned into pharmaceutical preparations for arbitrary routes of administration including parenteral, subcutaneous, intraperitoneal, intrapulmonary, and intranasal administrations, and, if necessary, for local therapy and intralesional administration. As the parenteral infusion, there may be mentioned intravenous, intraarterial, intraperitoneal, intramuscular, intracutaneous, or subcutaneous administration. The preparation is preferably administered by injection, and most preferably by intravenous or subcutaneous injection. The preferable administration includes local administration, particularly, percutaneous, transmucosal, rectum, and peroral administrations, or local administration by way of a catheter placed near a desired part, for example. The preparation may contain a pharmaceutically permissible excipient or diluent, according to the route of administration. As examples of such an excipient, there may be mentioned water, pharmaceutically allowable organic solvents.

For the aqueous suspension preparation or aqueous preparation of the present invention, an administrating device such as a prefilled syringe or a two-air-chamber prefilled syringe may be mentioned as an example. In the case of administering a protein for medical use, conventionally, intravenous drip, subcutaneous injection or the like has been performed, and administration by health care staff in a medical institution or self-administration at the patient's home has been conducted. In the case where the protein for medical use is insufficient in thermal stability, cold storage may be needed. Storing prefilled syringes for self-injection or the like in a refrigerator is a heavy burden on the patient. In the case where a syringe having two air chambers, for example, is used while holding a complex-containing aqueous suspension preparation of the present invention having thermal stability as depicted in Examples 102 to 106 described later in one of the chambers, and holding a low-concentration electrolyte in the other chamber, to thereby enhance shelf stability, it is considered possible to store the syringe at room temperature instead of cold storage. This, if realized, has a great merit. In using the syringe, the two air chambers are brought into fluid communication with each other, whereby the preparation can be administered as an aqueous preparation.

FIG. 8 depicts an example of the two-air-chamber prefilled syringe. A prefilled syringe 1 includes a sheath 2, gaskets 3 and 4 slidable within the sheath 2, and a plunger 5 (proximal side) for operation to move the gaskets 3 and 4. In a first chamber on the distal side which is surrounded by the sheath 2 and the gasket 3, a protein-polyamino acid complex 6 of the present invention is preliminarily accommodated in a liquid-tight manner. In a second chamber on the proximal side which is separated from the first chamber by the gasket 3, an aqueous electrolyte solution as a dissolving liquid 7 is accommodated and is sealed by the plunger 5 and the gaskets 3 and 4. The sheath 2 is provided with an enlarged diameter portion 8 at the first chamber. This state exists during storage. During storage, a protein for medical use of the present invention is preserved in the polyamino acid complex of the present invention, with high stability.

At the time of dissolution, the plunger 5 is pushed to make the gasket 4 push the dissolving liquid 7, so that the gasket 3 pushed by the dissolving liquid 7 moves to the distal side where the enlarged diameter portion 8 exists, the dissolving liquid 7 flows into the first chamber on the distal side by way of the enlarged diameter portion 8, and is mixed with the protein-polyamino acid complex 6 of the present invention, to re-dissolve the protein in the complex, thereby forming a protein solution.

### [Method of concentrating protein, method of producing aqueous preparation having concentrated, or equivalent, or diluted protein concentration]

(Step 1): The pH of a buffer is adjusted so that the pH is deviated to the basic side and/or the acidic side from the isoelectric point pI of the protein by an absolute value of, for example, 0.5 to 4.0.
(Step 2): To the buffer, the protein having a surface charge is added so as to attain a concentration in the range from 0.01 mg/mL to 50 mg/mL, and the polyamino acid is added so as to attain a concentration in the range from 0.01 mg/mL to 100 mg/mL, thereby forming an aqueous suspension which contains the protein-polyamino acid complex.
(Step 3): At least part of water or buffer is removed from the complex-containing aqueous suspension containing obtained after the step 1 and step 2, by a method selected from among centrifugation, ultrafiltration, supernatant removal and the like, thereby adjusting the aqueous suspension so that the protein is contained at a concentration in the range from 0.1 mg/mL to 500 mg/mL.
(Step 4): To the aqueous suspension prepared in the step 3, a low-concentration electrolyte in an amount smaller than the amount of water or buffer removed in the step 3 is added, so as to re-dissolve the protein in the complex into the buffer, thereby obtaining an aqueous liquid having a concentrated protein concentration.
   Alternatively, a low-concentration electrolyte in an amount equal to the amount of water or buffer removed in the step 3 is added, so as to re-dissolve the protein in the complex, thereby obtaining an aqueous liquid having an equivalent protein concentration. Yet alternatively, a low-concentration electrolyte in an amount larger than the amount of water or buffer removed in the step 3 is added, so as to re-dissolve the protein in the complex, thereby obtaining an aqueous liquid having a diluted protein concentration.
(Step 5): Without removing water from the complex-containing aqueous suspension obtained after the step 1 and step 2, a low-concentration electrolyte is added to the aqueous suspension, so as to re-dissolve the protein in the complex into the buffer, thereby obtaining an aqueous liquid having a diluted protein concentration.

In the case where the above-mentioned protein is a protein for medical use, an aqueous preparation is produced by the above step 1, step 2, step 3, and step 4, or by the above step 1, step 2, and step 5.

In the step 1, it is preferable to obtain the complex by adjusting the pH of the buffer so that |pI - pH| will be within the range from 0.5 to 4.0, where pI is the isoelectric point of the protein contained.

### (1) Method of concentrating protein

The following steps 1) to 3) can be conducted in an arbitrary order, and may be carried out simultaneously or sequentially.
1) A buffer is added to a protein.
2) A polyamino acid is added to the buffer.
3) The pH of the buffer is adjusted so that |pI - pH| will be not less than 0.5, where pI is the isoelectric point of the protein contained.
4) The protein and the polyamino acid form a complex in the buffer, whereby an aqueous suspension is obtained.
5) At least part of water or buffer is removed. A protein is obtained such that the concentration of the protein in the complex obtained is concentrated as compared to the concentration of the protein in the buffer in the step 1).
6) If necessary, the aqueous suspension obtained is centrifuged, to obtain the complex.

### (2) Method of dissolving complex

The complex obtained can be re-dissolved in the aqueous suspension by addition of a low-concentration electrolyte. As examples of the electrolyte, there can be mentioned NaCl, KCl, CaCl₂, MgCl₂ and the like.
Preferably, NaCl, which is the highest in biocompatibility, is selected, its concentration being not more than 5% by weight. The concentration of NaCl used for re-dissolution is preferably not less than 8 mM (0.048% by weight), more preferably not less than 40 mM (0.24% by weight).

In order to re-dissolve the obtained complex so as to obtain the protein dissolved in the buffer in a reversible amount, it is preferable to bring the |pI - pH| into the range from 0.5 to 4.0.

### (3) Protein concentration measuring method

The concentration of the protein in the buffer is calculated, for example, from a standard curve obtained by measuring absorbance at a wavelength of 280 nm. The concentration of the protein in the complex is calculated, for example, from a standard curve obtained by measuring absorbance at a wavelength of 280 nm after the complex obtained by centrifugation is dissolved by the method of (2) above. For instance, variation in concentration when the protein is concentrated is calculated in terms of concentration factor based on the non-concentrated. Besides, stress such as shaking, oxidation, heat or the like is loaded on the complex, and variation in concentration after the loading is calculated in terms of retention rate based on the concentration before the loading.

### (4) Method of measuring variation in secondary structure of protein

The CD spectrum of the protein in the complex is measured for an aqueous solution obtained by dissolving the protein by the method of (2) above. Stress such as shaking, oxidation, heat or the like is loaded on the complex, the CD spectrum is measured before the loading and after the loading, and variation in the secondary structure of the protein is detected. Similarly, for a control liquid of the protein, the CD spectrum is measured at the same concentration in the same buffer. If comparison of this spectrum with the CD spectrum after the loading of the complex does not depict any variation, it is depicted that the secondary structure of the protein in the complex has not been changed by the aforesaid loading.

### (5) Method of measuring activity of protein

### 1) Anti-IgE monoclonal antibody activity (IgE binding capacity or IgE receptor inhibitory capacity)

Measured in an aqueous solution. The complex is dissolved by the method of (2) above, and measurement is conducted. In regard of an anti-IgE monoclonal antibody, an activity for binding to IgE or an inhibitory activity on binding of IgE to an IgE receptor is measured by an ELISA method. Both of the activity measurements are assays for examining the binding between IgE and the anti-IgE antibody. In an IgE binding test, the concentration of the anti-IgE monoclonal antibody bound to IgE is measured. In an IgE receptor inhibitory test, the concentration of IgE bound to the IgE receptor is measured. For instance, variation in activity when the protein is concentrated is calculated in terms of multiplying factor of activity based on the activity when the protein is non-concentrated. Besides, stress such as shaking, oxidation, heat or the like is loaded on the complex, and variation in activity after the loading is calculated in terms of retention rate based on the value before the loading. Specifically, Test Example 4 in Example 74 is measured by an IgE binding test, and activities of other anti-IgE monoclonal antibodies are measured by a receptor inhibition test.

### 2) Anti-TNFα monoclonal antibody activity (TNFα binding capacity)

Measured in an aqueous solution. The complex is dissolved by the method of (2) above, and measurement is conducted. In regard of an anti-TNFα monoclonal antibody, activity for binding to TNFα is measured by an ELISA method. For example, variation in activity when the protein is concentrated is calculated in terms of multiplying factor of activity based on the activity when the protein is non-concentrated. Besides, stress such as shaking, oxidation, heat or the like is loaded on the complex, and variation in activity after the loading is calculated in terms of retention rate based on the value before the loading.

### 3) Anti-TNFα monoclonal antibody Fab fragment activity (TNFα binding inhibitory capacity)

Measured in an aqueous solution. The complex is dissolved by the method of (2) above, and measurement is conducted. In regard of an anti-TNFα monoclonal antibody Fab fragment, inhibitory activity on binding between an anti-TNFα monoclonal antibody and TNFα is measured by an ELISA method. For instance, variation in activity when the protein is concentrated is calculated in terms of multiplying factor based on the value when the protein is non-concentrated.

### (6) Method of measuring stability against shaking stress

### 1) Stability of anti-EGFR monoclonal antibody against shaking stress

Variation in protein content before and after shaking stress is measured as protein content retention rate. Before shaking stress, absorbance is measured, and concentration of an anti-EGFR monoclonal antibody is thereby measured. Shaking stress is given, then an insoluble precipitate in the case where insoluble aggregates are formed by the shaking stress is removed by centrifugation, after which the absorbance is measured to measure the concentration of the anti-EGFR monoclonal antibody, and protein rate content retention rate is determined.

### 2) Stability of anti-TNFα monoclonal antibody against shaking stress

Variation in protein content before and after shaking stress is measured as protein content retention rate. Before shaking stress, absorbance is measured, and concentration of an anti-TNFα monoclonal antibody is thereby measured. Shaking stress is given, then an insoluble precipitate in the case where insoluble aggregates are formed by the shaking stress is removed by centrifugation, after which the absorbance is measured to measure the concentration of the anti-TNFα monoclonal antibody, and protein content retention rate is determined.

### 3) Stability of anti-IgE monoclonal antibody against shaking stress

Variation in protein content before and after shaking is measured as protein content retention rate. Before shaking stress, absorbance is measured, and concentration of an anti-IgE monoclonal antibody is thereby measured. Shaking stress is given, then an insoluble precipitate in the case where insoluble aggregates are formed by the shaking stress is removed by centrifugation, after which absorbance is measured to measure the concentration of the anti-IgE monoclonal antibody, and protein content retention rate is determined.

### (7) Method of measuring rate of change in primary structure of protein

### 1) Peptide mapping method

A test method in which a protein is digested chemically or enzymatically, the resulting peptide fragments are separated and detected by liquid chromatography (LC) or the like, and, by comparison of chromatographic patterns obtained, variation in constituent amino acids is determined. The state of change in the primary structure of the protein can be calculated in terms of rate of change in the primary structure.

### (8) Method of measuring stability against thermal stress

### 1) Differential scanning calorimetry

A test method in which temperatures of a reference substance and a sample are measured while giving a predetermined heat to them, and an endothermic reaction or exothermic reaction due to a change in the state of the sample is measured. Input/output of heat attendant on a structural transition of the protein can be measured directly, and a thermal denaturing temperature of the protein can be measured.

### (9) Method of measuring aggregation inhibitory properties

1) Shaking stress is given to a complex of an anti-EGFR monoclonal antibody and to a control liquid in which not any complex is formed. In regard of samples before the stress and after the stress, the amount of insoluble aggregates precipitated upon centrifugation under the conditions to be specifically described in Examples later is determined as an amount obtained by subtracting the amount of the anti-EGFR monoclonal antibody remaining in the supernatant after the shaking from the total amount of the anti-EGFR monoclonal antibody before the shaking.
2) Shaking stress is given to a complex of anti-TNFα monoclonal antibody with polyglutamic acid and a control liquid in which not any complex is formed. In regard of samples before the shaking stress and after the shaking stress, the numbers of aggregates are each determined by a micro-flow imaging method.
3) Size exclusion chromatograph

Also called size exclusion chromatography, molecular sieve chromatography, and SEC (Size Exclusion Chromatography). A chromatography in which sifting based on the molecular size of a sample is used as a principle. Since protein molecules can diffuse into the inside of a carrier whereas aggregates cannot reach the inside of the carrier and flow away in the exterior of the carrier, the amount of the aggregates can be measured. In the measurement in Example 51 to be described later, the amount of soluble aggregates is measured as a peak area. Examples

The present invention will be described more in detail below by depicting Examples, but the present invention is not limited to these Examples.

### [1. Examples 1 to 30 and Comparative Examples 1 to 30 which depict concentratability by polyamino acid, and negative control Comparative Example 2-2 which depicts that concentration is impossible by sole use of amino acid other than polyamino acid]

### <Anti-TNFα monoclonal antibody>

### (Example 1)

In a 10 mM MOPS buffer (pH 5.5), anti-tumor necrosis factor (TNF)-α monoclonal antibody (pI: 8.7, MW: 150 kDa) was prepared so as to attain a concentration of 0.5 mg/mL, and poly-L-glutamic acid (MW: 3 kDa to 15 kDa) was added thereto in amounts of 0.05 to 2 parts by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 2)

In a 10 mM MOPS buffer (pH 5.5), anti-TNFα monoclonal antibody was prepared so as to attain a concentration of 0.5 mg/mL, and poly-DL-aspartic acid (MW: 2 kDa to 11 kDa) was added thereto in amounts of 0.05 to 1 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-DL-aspartic acid complex-containing aqueous suspensions.

### (Example 3)

In a 10 mM MOPS buffer (pH 5.5), anti-TNFα monoclonal antibody was prepared so as to attain a concentration of 0.5 mg/mL, and poly-L-glutamic acid (MW: 0.75 kDa to 5 kDa) was added thereto in amounts of 0.5 to 7 parts by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 4)

In a 10 mM Tris-HCl buffer (pH 8.2), anti-TNFα monoclonal antibody was prepared so as to attain a concentration of 0.5 mg/mL, and poly-L-glutamic acid (MW: 3 kDa to 15 kDa) was added thereto in amounts of 0.05 to 1 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 5)

The same operations as in Example 4 except for using a 10 mM MOPS buffer (pH 7.7) were conducted, to obtain anti-TNFα monoclonal antibody-poly-L-glutamic acid complex-containing aqueous suspensions.

### (Example 6)

The same operations as in Example 4 except for using a 10 mM MOPS buffer (pH 6.5) were conducted, to obtain anti-TNFα monoclonal antibody-poly-L-glutamic acid complex-containing aqueous suspensions.

### (Example 7)

In a 10 mM citrate buffer (pH 4.7), anti-TNFα monoclonal antibody was prepared so as to attain a concentration of 0.5 mg/mL, and poly-L-glutamic acid (MW: 3 kDa to 15 kDa) was added thereto in amounts of 0.1 to 1 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 8)

In a 10 mM MOPS buffer (pH 7.0), anti-TNFα monoclonal antibody was prepared so as to attain a concentration of 0.5 mg/mL, and poly-L-glutamic acid (MW: 15 kDa to 50 kDa) was added thereto in amounts of 0.01 to 1 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 9)

The same operations as in Example 8 except for using poly-L-glutamic acid (MW: 50 kDa to 100 kDa) were conducted, to obtain anti-TNFα monoclonal antibody-poly-L-glutamic acid complex-containing aqueous suspensions.

### (Example 10)

In a 10 mM phosphate buffer (pH 7.0), anti-TNFα monoclonal antibody was prepared so as to attain a concentration of 0.5 mg/mL, and poly-L-glutamic acid (MW: 3 kDa to 15 kDa) was added thereto in amounts of 0.05 to 0.5 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Example 11)

The same operations as in Example 10 except for using a 10 mM PIPES (piperazine-1,4-bis(2-ethanesulfonic acid)) buffer (pH 7.0) were conducted, to obtain anti-TNFα monoclonal antibody-poly-L-glutamic acid complex-containing aqueous suspensions.

### (Example 12)

The same operations as in Example 10 except for using a 10 mM MES buffer (pH 7.0) were conducted, to obtain anti-TNFα monoclonal antibody-poly-L-glutamic acid complex-containing aqueous suspensions.

### (Example 13)

The same operations as in Example 10 except for using a 10 mM HEPES buffer (pH 7.0) were conducted, to obtain anti-TNFα monoclonal antibody-poly-L-glutamic acid complex-containing aqueous suspensions.

### <Anti-IgE monoclonal antibody>

### (Example 14)

In a 10 mM MOPS buffer (pH 5.5), anti-immunoglobulin E (IgE) monoclonal antibody (pI: 7.6, MW: 150 kDa) was prepared so as to attain a concentration of 0.5 mg/mL, and poly-L-glutamic acid (MW: 3 kDa to 15 kDa) was added thereto in amounts of 0.025 to 0.5 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### (Example 15)

The same operations as in Example 14 except for using a 10 mM MOPS buffer (pH 6.5) were conducted, to obtain anti-IgE monoclonal antibody-poly-L-glutamic acid complex-containing aqueous suspensions.

### (Example 16)

In a 10 mM MOPS buffer (pH 5.5), anti-IgE monoclonal antibody was prepared so as to attain a concentration of 0.5 mg/mL, and poly-DL-aspartic acid (MW: 2 kDa to 11 kDa) was added thereto in amounts of 0.03 to 1 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-DL-aspartic acid complex-containing aqueous suspensions.

### (Example 17)

In a 10 mM MOPS buffer (pH 5.5), anti-IgE monoclonal antibody was prepared so as to attain a concentration of 15.0 mg/mL, and poly-L-glutamic acid (MW: 3 kDa to 15 kDa) was added thereto in amounts of 0.05 to 0.3 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### (Example 18)

In a 10 mM citrate buffer (pH 5.4), anti-IgE monoclonal antibody was prepared so as to attain a concentration of 0.5 mg/mL, and poly-L-glutamic acid (MW: 3 kDa to 15 kDa) was added thereto in amounts of 0.05 to 1 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### (Example 19)

In a 10 mM citrate buffer (pH 4.1), anti-IgE monoclonal antibody was prepared so as to attain a concentration of 0.5 mg/mL, and poly-L-glutamic acid (MW: 3 kDa to 15 kDa) was added thereto in amounts of 0.1 to 1 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### (Example 20)

The same operations as in Example 19 except for using a 10 mM citrate buffer (pH 3.6) were conducted, to obtain anti-IgE monoclonal antibody-poly-L-glutamic acid complex-containing aqueous suspensions.

### <Anti-EGFR monoclonal antibody>

### (Example 21)

In a 10 mM MOPS buffer (pH 5.0), anti-epidermal growth factor receptor (EGFR) monoclonal antibody (pI: 6.9, MW: 150 kDa) was prepared so as to attain a concentration of 1.0 mg/mL, and poly-L-glutamic acid (MW: 3 kDa to 15 kDa) was added thereto in amounts of 0.01 to 0.5 part by mass based on 1 part by mass of anti-EGFR monoclonal antibody. Each of these prepared liquids was centrifuged, and 90% of the total volume of the centrifuged liquid was removed as supernatant, to obtain anti-EGFR monoclonal antibody-poly-L-glutamic acid complex-containing aqueous suspensions.

### (Example 22)

The same operations as in Example 21 except for using a 10 mM MOPS buffer (pH 5.5) were conducted, to obtain anti-EGFR monoclonal antibody-poly-L-glutamic acid complex-containing aqueous suspensions.

### (Example 23)

In a 10 mM citrate buffer (pH 4.7), anti-EGFR monoclonal antibody was prepared so as to attain a concentration of 0.5 mg/mL, and poly-L-glutamic acid (MW: 3 kDa to 15 kDa) was added thereto in amounts of 0.05 to 1 part by mass based on 1 part by mass of anti-EGFR monoclonal antibody. Each of these prepared liquids was centrifuged, and 90% of the total volume of the centrifuged liquid was removed as supernatant, to obtain anti-EGFR monoclonal antibody-poly-L-glutamic acid complex-containing aqueous suspensions.

### (Example 24)

The same operations as in Example 23 except for using a 10 mM MES buffer (pH 4.7) were conducted, to obtain anti-EGFR monoclonal antibody-poly-L-glutamic acid complex-containing aqueous suspensions.

### (Example 25)

In a 10 mM citrate buffer (pH 3.4), anti-EGFR monoclonal antibody was prepared so as to attain a concentration of 0.5 mg/mL, and poly-L-glutamic acid (MW: 3 kDa to 15 kDa) was added thereto in amounts of 0.3 to 1 part by mass based on 1 part by mass of anti-EGFR monoclonal antibody. Each of these prepared liquids was centrifuged, and 90% of the total volume of the centrifuged liquid was removed as supernatant, to obtain anti-EGFR monoclonal antibody-poly-L-glutamic acid complex-containing aqueous suspensions.

### (Example 26)

In a 10 mM citrate buffer (pH 2.9), anti-EGFR monoclonal antibody was prepared so as to attain a concentration of 0.5 mg/mL, and poly-L-glutamic acid (MW: 3 kDa to 15 kDa) was added thereto in amounts of 0.5 to 1.5 parts by mass based on 1 part by mass of anti-EGFR monoclonal antibody. Each of these prepared liquids was centrifuged, and 90% of the total volume of the centrifuged liquid was removed as supernatant, to obtain anti-EGFR monoclonal antibody-poly-L-glutamic acid complex-containing aqueous suspensions.

### <Anti-HER2 monoclonal antibody>

### (Example 27)

In a 10 mM MOPS buffer (pH 6.5), anti-human epidermal growth factor receptor (HER) 2 monoclonal antibody (pI: 8.7, MW: 150 kDa) was prepared so as to attain a concentration of 0.5 mg/mL, and poly-L-glutamic acid (MW: 3 kDa to 15 kDa) was added thereto in amounts of 0.05 to 1 part by mass based on 1 part by mass of anti-HER2 monoclonal antibody. Each of these prepared liquids was centrifuged, and 90% of the total volume of the centrifuged liquid was removed as supernatant, to obtain anti-HER2 monoclonal antibody-poly-L-glutamic acid complex-containing aqueous suspensions.

### (Example 28)

The same operations as in Example 27 except for using a 10 mM MOPS buffer (pH 7.7) were conducted, to obtain anti-HER2 monoclonal antibody-poly-L-glutamic acid complex-containing aqueous suspensions.

### (Example 29)

The same operations as in Example 27 except for using a 10 mM Tris-HCl buffer (pH 8.2) were conducted, to obtain anti-HER2 monoclonal antibody-poly-L-glutamic acid complex-containing aqueous suspensions.

### (Example 30)

In a 10 mM citrate buffer (pH 4.7), anti-HER2 monoclonal antibody was prepared so as to attain a concentration of 0.5 mg/mL, and poly-L-glutamic acid (MW: 3 kDa to 15 kDa) was added thereto in amounts of 0.1 to 1 part by mass based on 1 part by mass of anti-HER2 monoclonal antibody. Each of these prepared liquids was centrifuged, and 90% of the total volume of the centrifuged liquid was removed as supernatant, to obtain anti-HER2 monoclonal antibody-poly-L-glutamic acid complex-containing aqueous suspensions.

### (Comparative Examples 1 to 30)

The same operations as in Examples 1 to 30 except for not adding the polyamino acid in Examples 1 to 30 were conducted.

### [Test Example 1]

In regard of Examples 1 to 30 above, sodium chloride was added to each of the above-obtained protein-polyamino acid complex-containing aqueous suspension so as to attain a concentration of 150 mM, and absorbance at a wavelength of 280 nm was measured, whereby the protein concentration was measured. In addition, in regard of Comparative Examples 1 to 30 above, absorbance at a wavelength of 280 nm was measured, whereby the protein concentration was measured. From these results, the ratio of the protein concentration in each Example to the protein concentration in each Comparative Examples was determined. In all of Examples 1 to 30, a rise in protein concentration was found, which depicted that the protein can be concentrated. Details of the results are set forth in Tables 1 to 4.

**[Table 1]**

| | | Comparative Example | Example | | | | | | |
|---|---|---|---|---|---|---|---|---|---|
| Example 1 | Polyamino acid/protein mass ratio | 0 | 0.05 | 0.1 | 0.2 | 0.3 | 0.5 | 1 | 2 |
| | Protein concentration ratio | 1 | 4.6 | 10.3 | 10.4 | 8.3 | 7.5 | 7.4 | 4.9 |
| Example 2 | Polyamino acid/protein mass ratio | 0 | 0.05 | 0.1 | 0.15 | 0.3 | 0.5 | 0.7 | 1 |
| | Protein concentration ratio | 1 | 10.7 | 9.6 | 7.6 | 4.4 | 3.7 | 2.8 | 1.1 |
| Example 3 | Polyamino acid/protein mass ratio | 0 | 0.5 | 1 | 1.5 | 2 | 2.5 | 3 | 3.5 |
| | Protein concentration ratio | 1 | 9.4 | 10.3 | 10.1 | 10 | 9.3 | 8.8 | 8 |
| | Polyamino acid/protein mass ratio | | 4 | 4.5 | 5 | 5.5 | 6 | 6.5 | 7 |
| | Protein concentration ratio | | 7.1 | 5.9 | 4.4 | 2.9 | 1.9 | 1.3 | 1.1 |
| Example 4 | Polyamino acid/protein mass ratio | 0 | 0.05 | 0.1 | 0.3 | 0.5 | 1 | | |
| | Protein concentration ratio | 1 | 8.7 | 8.3 | 5.1 | 3.4 | 0.9 | | |

**[Table 2]**

| | | Comparative Example | Example | | | | | | |
|---|---|---|---|---|---|---|---|---|---|
| Example 5 | Polyamino acid/protein mass ratio | 0 | 0.05 | 0.1 | 0.3 | 0.5 | 1 | | |
| | Protein concentration ratio | 1 | 9.5 | 9.4 | 8.4 | 7.6 | 5 | | |
| Example 6 | Polyamino acid/protein mass ratio | 0 | 0.05 | 0.1 | 0.3 | 0.5 | 1 | | |
| | Protein concentration ratio | 1 | 10 | 9.6 | 8.9 | 8.9 | 6.4 | | |
| Example 7 | Polyamino acid/protein mass ratio | 0 | 0.1 | 0.3 | 0.5 | 1 | | | |
| | Protein concentration ratio | 1 | 7.1 | 9.7 | 10.1 | 9.9 | | | |
| Example 8 | Polyamino acid/protein mass ratio | 0 | 0.01 | 0.025 | 0.05 | 0.075 | 0.1 | 0.15 | 0.3 |
| | Protein concentration ratio | 1 | 5.9 | 10 | 9.9 | 9.9 | 9.7 | 8.7 | 6.9 |
| | Polyamino acid/protein mass ratio | | 0.5 | 1 | | | | | |
| | Protein concentration ratio | | 5.4 | 4.8 | | | | | |
| Example 9 | Polyamino acid/protein mass ratio | 0 | 0.01 | 0.025 | 0.05 | 0.075 | 0.1 | 0.15 | 0.3 |
| | Protein concentration ratio | 1 | 5.8 | 9.9 | 9.1 | 8 | 6.8 | 5.1 | 1.9 |
| | Polyamino acid/protein mass ratio | | 0.5 | 1 | | | | | |
| | Protein concentration ratio | | 1 | 0.9 | | | | | |
| Example 10 | Polyamino acid/protein mass ratio | 0 | 0.05 | 0.1 | 0.2 | 0.3 | 0.5 | | |
| | Protein concentration ratio | 1 | 8.3 | 8.6 | 8.5 | 8.3 | 7.5 | | |
| Example 11 | Polyamino acid/protein mass ratio | 0 | 0.05 | 0.1 | 0.2 | 0.3 | 0.5 | | |
| | Protein concentration ratio | 1 | 8.2 | 9 | 8.8 | 8.7 | 8.1 | | |
| Example 12 | Polyamino acid/protein mass ratio | 0 | 0.05 | 0.1 | 0.2 | 0.3 | 0.5 | | |
| | Protein concentration ratio | 1 | 7.4 | 9.2 | 8.9 | 8.5 | 8.2 | | |
| Example 13 | Polyamino acid/protein mass ratio | 0 | 0.05 | 0.1 | 0.2 | 0.3 | 0.5 | | |
| | Protein concentration ratio | 1 | 9.3 | 9.1 | 8.6 | 7.8 | 6.2 | | |
| Example 14 | Polyamino acid/protein mass ratio | 0 | 0.025 | 0.05 | 0.1 | 0.15 | 0.3 | 0.5 | |
| | Protein concentration ratio | 1 | 3.3 | 7 | 10 | 10.2 | 6.1 | 0.9 | |
| Example 15 | Polyamino acid/protein mass ratio | 0 | 0.025 | 0.05 | 0.1 | 0.15 | 0.3 | 0.5 | |
| | Protein concentration ratio | 1 | 5.7 | 7.3 | 2.5 | 0.6 | 0.7 | 0.8 | |

**[Table 3]**

| | | Comparative Example | Example | | | | | | |
|---|---|---|---|---|---|---|---|---|---|
| Example 16 | Polyamino acid/protein mass ratio | 0 | 0.03 | 0.05 | 0.1 | 0.15 | 0.3 | 0.5 | 1 |
| | Protein concentration ratio | 1 | 4.9 | 10.2 | 10.2 | 7.5 | 1 | 1.1 | 1.1 |
| Example 17 | Polyamino acid/protein mass ratio | 0 | 0.05 | 0.1 | 0.15 | 0.3 | | | |
| | Protein concentration ratio | 1 | 10.3 | 9.7 | 7.5 | 1.8 | | | |
| Example 18 | Polyamino acid/protein mass ratio | 0 | 0.05 | 0.1 | 0.3 | 0.5 | 1 | | |
| | Protein concentration ratio | 1 | 10.1 | 10.4 | 9.8 | 9.6 | 9.4 | | |
| Example 19 | Polyamino acid/protein mass ratio | 0 | 0.1 | 0.3 | 0.5 | 1 | | | |
| | Protein concentration ratio | 1 | 3.9 | 10.5 | 10.7 | 10.6 | | | |
| Example 20 | Polyamino acid/protein mass ratio | 0 | 0.1 | 0.3 | 0.5 | 1 | | | |
| | Protein concentration ratio | 1 | 0.9 | 6.3 | 9.4 | 9.4 | | | |
| Example 21 | Polyamino acid/protein mass ratio | 0 | 0.01 | 0.025 | 0.05 | 0.1 | 0.15 | 0.3 | 0.5 |
| | Protein concentration ratio | 1 | 0.9 | 2.2 | 8.6 | 4.8 | 0.9 | 0.8 | 0.9 |
| Example 22 | Polyamino acid/protein mass ratio | 0 | 0.1 | 0.025 | 0.05 | 0.1 | 0.15 | 0.3 | 0.5 |
| | Protein concentration ratio | 1 | 1 | 3.9 | 8 | 2.5 | 0.9 | 0.8 | 0.9 |
| Example 23 | Polyamino acid/protein mass ratio | 0 | 0.05 | 0.1 | 0.3 | 0.5 | 1 | | |
| | Protein concentration ratio | 1 | 1.4 | 3.1 | 7.6 | 7.5 | 7.1 | | |

**[Table 4]**

| | | Comparative Example | Example | | | | | | |
|---|---|---|---|---|---|---|---|---|---|
| Example 24 | Polyamino acid/protein mass ratio | 0 | 0.05 | 0.1 | 0.15 | 0.3 | 0.5 | 1 | |
| | Protein concentration ratio | 1 | 3 | 9.2 | 10 | 9.5 | 6.5 | 0.9 | |
| Example 25 | Polyamino acid/protein mass ratio | 0 | 0.3 | 0.5 | 1 | | | | |
| | Protein concentration ratio | 1 | 5.1 | 9.8 | 9.8 | | | | |
| Example 26 | Polyamino acid/protein mass ratio | 0 | 0.5 | 1 | 1.25 | 1.5 | | | |
| | Protein concentration ratio | 1 | 1.7 | 9.3 | 9.3 | 9.5 | | | |
| Example | Polyamino acid/protein mass | 0 | 0.05 | 0.15 | 0.5 | 1 | | | |
| 27 | ratio | | | | | | | | |
| | Protein concentration ratio | 1 | 9.6 | 8.6 | 1.1 | 0.7 | | | |
| Example 28 | Polyamino acid/protein mass ratio | 0 | 0.05 | 0.15 | 0.5 | 1 | | | |
| | Protein concentration ratio | 1 | 8.2 | 4.8 | 0.7 | 0.7 | | | |
| Example 29 | Polyamino acid/protein mass ratio | 0 | 0.05 | 0.15 | 0.5 | 1 | | | |
| | Protein concentration ratio | 1 | 5.4 | 0.7 | 0.6 | 0.8 | | | |
| Example 30 | Polyamino acid/protein mass ratio | 0 | 0.1 | 0.3 | 0.5 | 1 | | | |
| | Protein concentration ratio | 1 | 6.4 | 9.2 | 9.3 | 9.4 | | | |

### (Comparative Example 2-2)

The same operations as in Example 2, except for using aspartic acid in place of poly-DL-aspartic acid (MW: 2 kDa to 11 kDa) in Example 2, were conducted as a negative control (Comparative Example 2-2).

### (Comparative Example 2)

As the negative control comparative example, the same sample as in Comparative Example 2, except for adding neither an amino acid nor a polyamino acid, was produced.

### [Test Example 2]

In regard of Comparative Example 2-2, sodium chloride was added to each of the obtained protein-amino acid-containing aqueous suspensions, so as to attain a concentration of 150 mM, and absorbance at a wavelength of 280 nm was measured, whereby the protein concentration was measured. Besides, in regard of Comparative Example 2, absorbance at a wavelength of 280 nm was measured, whereby the protein concentration was measured. From these results, the ratio of protein concentration in each Example to protein concentration in each Comparative Example was determined. In negative-control Comparative Example 2-2, a rise in protein concentration was not observed; thus, it was depicted that by the sole use of an amino acid which is not a polyamino acid, it is impossible to concentrate the protein. Details of the results were set forth in Table 5, together with the results of Example 2 in which concentration of the protein was possible by a polyamino acid.

**[Table 5]**

| | | Comparative Example | | | | | | |
|---|---|---|---|---|---|---|---|---|
| Example 2 | Polyamino acid/protein mass ratio | 0 | 0.05 | 0.1 | 0.15 | 0.3 | 0.5 | 1 |
| | Protein concentration ratio | 1 | 10.7 | 9.6 | 7.6 | 4.4 | 3.7 | 1.1 |
| Comparative Example 2-2 (negative control) | Amino acid/protein mass ratio | 0 | 0.05 | 0.1 | 0.15 | 0.3 | 0.5 | 1 |
| | Protein concentration ratio | 1 | 1 | 1 | 1 | 1 | 1 | 1 |

### [2. Examples 31 to 33 (other proteins) which depict possibility of concentration by polyamino acid]

### <High-concentration anti-TNFα monoclonal antibody>

### (Example 31)

In a 10 mM MOPS buffer (pH 6.5), anti-TNFα monoclonal antibody was prepared so as to attain a concentration of 15.0 mg/mL, and poly-L-glutamic acid (MW: 3 kDa to 15 kDa) was added thereto in amounts of 0.02 to 0.5 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### <High-concentration IgE monoclonal antibody>

### (Example 32)

In a 10 mM citrate buffer (pH 5.5), anti-IgE monoclonal antibody was prepared so as to attain a concentration of 15 mg/mL, and poly-L-glutamic acid (MW: 3 kDa to 15 kDa) was added thereto in amounts of 0.02 to 0.5 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### <Anti-TNFα monoclonal antibody Fab fragment>

### (Example 33)

In a 10 mM MOPS buffer (pH 6.5), tumor necrosis factor (TNF) α monoclonal antibody Fab fragment (pI: 8.8, MW: 110 kDa) was prepared so as to attain a concentration of 1.16 mg/mL, and poly-L-glutamic acid (MW: 3 kDa to 15 kDa) was added thereto in amounts of 0.025 to 0.5 part by mass based on 1 part by mass of anti-TNFα monoclonal antibody Fab fragment. Each of these prepared liquids was centrifuged, and 90% of the total volume of the centrifuged liquid was removed as supernatant, to obtain anti-TNFα monoclonal antibody Fab fragment-poly-L-glutamic acid complex-containing aqueous suspensions.

**[Table 6]**

| | | Comparative Example | | | | | | |
|---|---|---|---|---|---|---|---|---|
| Example 31 | Polyamino acid/protein mass ratio | 0 | 0.02 | 0.05 | 0.1 | 0.3 | 0.5 | |
| | Protein concentration ratio | 1 | 7.7 | 10.0 | 10.0 | 8.3 | 1.1 | |
| Example 32 | Polyamino acid/protein mass ratio | 0 | 0.02 | 0.05 | 0.1 | 0.3 | 0.5 | |
| | Protein concentration ratio | 1 | 9.9 | 10.1 | 10.2 | 9.9 | 8.8 | |
| Example 33 | Polyamino acid/protein mass ratio | 0 | 0.025 | 0.05 | 0.1 | 0.15 | 0.3 | 0.5 |
| | Protein concentration ratio | 1 | 8.8 | 9.8 | 8.6 | 7.7 | 4.5 | 2.7 |

### [3. Concentration of protein has maximum concentration factor]

In the method of concentrating a protein through forming a complex according to the present invention, it is seen that when concentration factor is tabulated in relation to pI and pH of the buffer used, the table depicts maximum concentration factors.

Referring to Table 7 below, the pH of a buffer was varied in relation to isoelectric point pI of each of proteins being concentrated in Tables 1 to 4, and, in a condition where the absolute value (|pI - pH|) of pI - pH is at the value as depicted in Table 7, the protein and a polyamino acid were added to the buffer to form a complex of the protein and the polyamino acid. The concentration of the protein in the complex obtained was measured, and the value of |pI - pH| at which a maximum value of the concentration factor is obtained was depicted.

Plotting the concentration factor against |pI - pH| gives the graphs depicted in FIG. 1. FIG. 1 depicts that a maximum concentration factor for the protein in the complex is present where |pI - pH| is in the range from 1.5 to 4.0.

The values of |pI - pH| at which the maximum values of concentration factor can be obtained are as follows. Anti-TNFα monoclonal antibody-poly-L-glutamic acid: 2.2 Anti-IgE monoclonal antibody-poly-L-glutamic acid: 2.2 Anti-EGFR monoclonal antibody-poly-L-glutamic acid: 2.2

**[Table 7]**

| Protein | Concentration factor | pI-pH | Buffer | | Polyglutamic acid |
|---|---|---|---|---|---|
| | | | Kind | pH | |
| Anti-TNFα antibody | | 0.5 | Tris-HCl | 8.2 | Polyglutamic acid |
| Anti-TNFα antibody | | 1.0 | Tris-HCl | 7.7 | Polyglutamic acid |
| Anti-TNFα antibody | Maximum | 2.2 | MOPS | 6.5 | Polyglutamic acid |
| Anti-TNFα antibody | | 3.5 | MOPS | 5.2 | Polyglutamic acid |
| Anti-TNFα antibody | | 4 | Citrate | 4.7 | Polyglutamic acid |
| Anti-IgE antibody | | 4.0 | Citrate | 3.6 | Polyglutamic acid |
| Anti-IgE antibody | | 3.5 | Citrate | 4.1 | Polyglutamic acid |
| Anti-IgE antibody | Maximum | 2.2 | Citrate | 5.4 | Polyglutamic acid |
| Anti-IgE antibody | | 1.0 | MOPS | 6.6 | Polyglutamic acid |
| Anti-IgE antibody | | 0.5 | MOPS | 7.1 | Polyglutamic acid |
| Anti-EGFR antibody | | 4.0 | Citrate | 2.9 | Polyglutamic acid |
| Anti-EGFR antibody | | 3.5 | Citrate | 3.4 | Polyglutamic acid |
| Anti-EGFR antibody | Maximum | 2.2 | Citrate | 4.7 | Polyglutamic acid |
| Anti-EGFR antibody | | 1.9 | Citrate | 5.0 | Polyglutamic acid |
| Anti-EGFR antibody | | 1.0 | Citrate | 5.9 | Polyglutamic acid |
| Anti-EGFR antibody | | 0.5 | MOPS | 6.4 | Polyglutamic acid |

### [4. Examples 34 to 41 which depict that activity is not damaged by concentration of protein]

### <Anti-IgE monoclonal antibody>

### (Example 34)

In a 10 mM MOPS buffer (pH 5.5), anti-IgE monoclonal antibody was prepared so as to attain a concentration of 0.5 mg/mL, and poly-L-glutamic acid (MW: 3 kDa to 15 kDa) was added thereto in amounts of 0.25, 0.05, 0.1, and 0.15 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspensions.

### (Comparative Example 34)

The same operations as in Example 34, except for not adding poly-L-glutamic acid, were conducted.

### [Test Example 3]

In regard of Example 34 above, sodium chloride was added to the obtained anti-IgE monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-IgE monoclonal antibody was measured. Besides, in regard of Comparative Example 34, absorbance at a wavelength of 280 nm was measured as measurement of protein concentration, and, further, the activity of anti-IgE monoclonal antibody was measured. From these results, the ratio of the protein concentration in Example 34 to the protein concentration in Comparative Example 34, and the ratio of the anti-IgE monoclonal antibody activity in Example 34to the anti-IgE monoclonal antibody activity in Comparative Example 34 were determined. It was found that both the protein concentration ratio and the activity ratio rose to about 10 times, depicting that the anti-IgE monoclonal antibody can be concentrated without damaging the activity thereof. The results were set forth in Table 8.

**[Table 8]**

| | | Comparative Example 34 | Example 34 | | | |
|---|---|---|---|---|---|---|
| Test Example 3 | Polyamino acid/protein mass ratio | 0 | 0.025 | 0.05 | 0.1 | 0.15 |
| | Protein concentration ratio | 1 | 5.4 | 9.2 | 10.2 | 9.1 |
| | Activity ratio | 1 | 5.5 | 9 | 9.6 | 9.1 |

### (Example 35)

In a 10 mM MOPS buffer (pH 5.5), anti-IgE monoclonal antibody was prepared so as to attain a concentration of 15 mg/mL, and poly-L-glutamic acid (MW: 3 kDa to 15 kDa) was added thereto in amounts of 0.05, 0.10, and 0.15 part by mass based on 1 part by mass of anti-IgE monoclonal antibody. Each of these prepared liquids was centrifuged, and 90% of the total volume of the centrifuged liquid was removed as supernatant, to obtain anti-IgE monoclonal antibody-poly-L-glutamic acid complex-containing aqueous suspension.

### (Comparative Example 35)

The same operations as in Example 35, except for not adding poly-L-glutamic acid, were conducted.

### [Test Example 4]

In regard of Example 35 above, sodium chloride was added to the obtained anti-IgE monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-IgE monoclonal antibody was measured. Besides, in regard of Comparative Example 35, absorbance at a wavelength of 280 nm was measured as measurement of protein concentration, and, further, the activity of anti-IgE monoclonal antibody was measured. From the results of these measurements, the ratio of the protein concentration in Example 35 to the protein concentration in Comparative Example 35, and the ratio of the anti-IgE monoclonal antibody activity in Example 35to the anti-IgE monoclonal antibody activity in Comparative Example 35 were determined. It was found that both the protein concentration ratio and the activity ratio rose to about 10 times, depicting that the anti-IgE monoclonal antibody can be concentrated without damaging the activity thereof. The results were set forth in Table 9.

**[Table 9]**

| | | Comparative Example 35 | Example 35 | | |
|---|---|---|---|---|---|
| Test Example 4 | Polyamino acid/protein mass ratio | 0 | 0.05 | 0.1 | 0.15 |
| | Protein concentration ratio | 1 | 10.3 | 9.7 | 7.5 |
| | Activity ratio | 1 | 10.5 | 10.2 | 7.3 |

### (Example 36)

In a 10 mM citrate buffer (pH 5.5), anti-IgE monoclonal antibody was prepared so as to attain a concentration of 15 mg/mL, and poly-L-glutamic acid (MW: 3 kDa to 15 kDa) was added thereto in an amount of 0.05 part by mass based on 1 part by mass of anti-IgE monoclonal antibody. Each of these prepared liquids was centrifuged, and 90% of the total volume of the centrifuged liquid was removed as supernatant, to obtain an anti-IgE monoclonal antibody-poly-L-glutamic acid complex-containing aqueous suspension.

### (Comparative Example 36)

The same operations as in Example 36, except for not adding poly-L-glutamic acid, were conducted.

### [Test Example 5]

In regard of Example 36 above, sodium chloride was added to the obtained anti-IgE monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-IgE monoclonal antibody was measured. Besides, in regard of Comparative Example 36, absorbance at a wavelength of 280 nm was measured as measurement of protein concentration, and, further, the activity of anti-IgE monoclonal antibody was measured. From the results of these measurements, the ratio of the protein concentration in Example 36 to the protein concentration in Comparative Example 36, and the ratio of the anti-IgE monoclonal antibody activity in Example 36 to the anti-IgE monoclonal antibody activity in Comparative Example 36 were determined. It was found that both the protein concentration ratio and the activity ratio rose to about 10 times, depicting that the anti-IgE monoclonal antibody can be concentrated without damaging the activity thereof. The results were set forth in Table 10.

**[Table 10]**

| | | Comparative Example 36 | Example 36 |
|---|---|---|---|
| Test Example 5 | Polyamino acid/protein mass ratio | 0 | 0.5 |
| | Protein concentration ratio | 1 | 10.1 |
| | Activity ratio | 1 | 10.6 |

### (Example 37)

In a 10 mM citrate buffer (pH 3.6), anti-IgE monoclonal antibody was prepared so as to attain a concentration of 0.5 mg/mL, and poly-L-glutamic acid (MW: 3 kDa to 15 kDa) was added thereto in an amount of 0.6 part by mass based on 1 part by mass of anti-IgE monoclonal antibody. Each of these prepared liquids was centrifuged, and 90% of the total volume of the centrifuged liquid was removed as supernatant, to obtain each anti-IgE monoclonal antibody-poly-L-glutamic acid complex-containing aqueous suspension.

### (Comparative Example 37)

The same operations as in Example 37, except for not adding poly-L-glutamic acid, were conducted.

### [Test Example 6]

In regard of Example 37 above, sodium chloride was added to each of the obtained anti-IgE monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-IgE monoclonal antibody was measured. Besides, in regard of Comparative Example 37, absorbance at a wavelength of 280 nm was measured as measurement of protein concentration, and, further, the activity of anti-IgE monoclonal antibody was measured. From the results of these measurements, the ratio of the protein concentration in Example 37 to the protein concentration in Comparative Example 37, and the ratio of the anti-IgE monoclonal antibody activity in Example 37 to the anti-IgE monoclonal antibody activity in Comparative Example 37 were determined. It was found that both the protein concentration ratio and the activity ratio rose to about 10 times, depicting that the anti-IgE monoclonal antibody can be concentrated without damaging the activity thereof. The results were set forth in Table 11.

**[Table 11]**

| | | Comparative Example 37 | Example 37 |
|---|---|---|---|
| Test Example 6 | Polyamino acid/protein mass ratio | 0 | 0.6 |
| | Protein concentration ratio | 1 | 10.1 |
| | Activity ratio | 1 | 10.2 |

### <Anti-TNFα monoclonal antibody>

### (Example 38)

In a 10 mM MOPS buffer (pH 6.5), anti-TNFα monoclonal antibody was prepared so as to attain a concentration of 0.5 mg/mL, and poly-L-glutamic acid (MW: 3 kDa to 15 kDa) was added thereto in amounts of 0.05, 0.1, 0.15, 0.3, 0.5, and 1 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Comparative Example 38)

The same operations as in Example 38, except for not adding poly-L-glutamic acid, were conducted.

### [Test Example 9]

In regard of Example 38 above, sodium chloride was added to the obtained anti-TNFα monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-TNFα monoclonal antibody was measured. Besides, in regard of Comparative Example 38, absorbance at a wavelength of 280 nm was measured as measurement of protein concentration, and, further, the activity of anti-TNFα monoclonal antibody was measured. From the results of these measurements, the ratio of the protein concentration in Example 38 to the protein concentration in Comparative Example 38, and the ratio of the anti-TNFα monoclonal antibody activity in Example 38 to the anti-TNFα monoclonal antibody activity in Comparative Example 38 were determined. It was found that both the protein concentration ratio and the activity ratio rose to about 10 times, depicting that the anti-TNFα monoclonal antibody can be concentrated without damaging the activity thereof. The results were set forth in Table 12.

**[Table 12]**

| | | Comparative Example 38 | Example 38 | | | | |
|---|---|---|---|---|---|---|---|
| Test Example 9 | Polyamino acid/protein mass ratio | 0 | 0.05 | 0.1 | 0.3 | 0.5 | 1 |
| | Protein concentration ratio | 1 | 10.2 | 10.2 | 9 | 7.7 | 6.4 |
| | Activity ratio | 1 | 10.3 | 10.3 | 8.5 | 7.1 | 5.8 |

### (Example 39)

In a 10 mM MOPS buffer (pH 6.5), anti-TNFα monoclonal antibody was prepared so as to attain a concentration of 15 mg/mL, and poly-L-glutamic acid (MW: 3 kDa to 15 kDa) was added thereto in an amount of 0.05 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody-poly-L-glutamic acid complex-containing aqueous suspensions.

### (Comparative Example 39)

The same operations as in Example 39, except for not adding poly-L-glutamic acid, were conducted.

### [Test Example 10]

In regard of Example 39 above, sodium chloride was added to the obtained anti-TNFα monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-TNFα monoclonal antibody was measured. Besides, in regard of Comparative Example 39, absorbance at a wavelength of 280 nm was measured as measurement of protein concentration, and, further, the activity of anti-TNFα monoclonal antibody was measured. From the results of these measurements, the ratio of the protein concentration in Example 39 to the protein concentration in Comparative Example 39, and the ratio of the anti-TNFα monoclonal antibody activity in Example 39 to the anti-TNFα monoclonal antibody activity in Comparative Example 39 were determined. It was found that both the protein concentration ratio and the activity ratio rose to about 10 times, depicting that the anti-TNFα monoclonal antibody can be concentrated without damaging the activity thereof. The results were set forth in Table 13.

**[Table 13]**

| | | Comparative Example 39 | Example 39 |
|---|---|---|---|
| Test Example 10 | Polyamino acid/protein mass ratio | 0 | 0.05 |
| | Protein concentration ratio | 1 | 10.0 |
| | Activity ratio | 1 | 10.6 |

### (Example 40)

In a 10 mM citrate buffer (pH 4.7), anti-TNFα monoclonal antibody was prepared so as to attain a concentration of 0.5 mg/mL, and poly-L-glutamic acid (MW: 3 kDa to 15 kDa) was added thereto in an amount of 0.4 part by mass based on 1 part by mass of anti-TNFα monoclonal antibody. Each of these prepared liquids was centrifuged, and 90% of the total volume of the centrifuged liquid was removed as supernatant, to obtain each anti-TNFα monoclonal antibody-poly-L-glutamic acid complex-containing aqueous suspensions.

### (Comparative Example 40)

The same operations as in Example 40, except for not adding poly-L-glutamic acid, were conducted.

### [Test Example 11]

In regard of Example 40 above, sodium chloride was added to each of the obtained anti-TNFα monoclonal antibody-poly-L-glutamic acid complex-containing aqueous suspension so as to attain a concentration of 150 mM, then absorbance at a wavelength of 280 nm was measured as measurement of protein concentration, and, further, the activity of anti-TNFα monoclonal antibody was measured. Besides, in regard of Comparative Example 40, absorbance at a wavelength of 280 nm was measured as measurement of protein concentration, and, further, the activity of anti-TNFα monoclonal antibody was measured. From these results, the ratio of the protein concentration in Example 40 to the protein concentration in Comparative Example 40, and the ratio of the anti-TNFα monoclonal antibody activity in Example 40 to the anti-TNFα monoclonal antibody activity in Comparative Example 40 were determined. It was found that both the protein concentration ratio and the activity ratio rose to about 10 times, depicting that the anti-TNFα monoclonal antibody can be concentrated without damaging the activity thereof. The results were set forth in Table 14.

**[Table 14]**

| | | Comparative Example 40 | Example 40 |
|---|---|---|---|
| Test Example 11 | Polyamino acid/protein mass ratio | 0 | 0.4 |
| | Protein concentration ratio | 1 | 10.4 |
| | Activity ratio | 1 | 10.2 |

### <Anti-TNFα monoclonal antibody Fab fragment>

### (Example 41)

In a 10 mM MOPS buffer (pH 6.5), anti-tumor necrosis factor (TNF) α monoclonal antibody Fab fragment (pI: 8.8, MW: 110 kDa) was prepared so as to attain a concentration of 1.16 mg/mL, and poly-L-glutamic acid (MW: 3 kDa to 15 kDa) was added thereto in amounts of 0.05, 0.1, and 0.3 part by mass based on 1 part by mass of anti-TNFα monoclonal antibody. Each of these prepared liquids was centrifuged, and 90% of the total volume of the centrifuged liquid was removed as supernatant, to obtain anti-TNFα monoclonal antibody Fab fragment-poly-L-glutamic acid complex-containing aqueous suspensions.

### [Test Example 12]

In regard of Example 41 above, sodium chloride was added to the obtained anti-TNFα monoclonal antibody Fab fragment-poly-L-glutamic acid complex-containing aqueous suspension so as to attain a concentration of 150 mM, then absorbance at a wavelength of 280 nm was measured as measurement of protein concentration, and, further, the activity of anti-TNFα monoclonal antibody Fab fragment was measured. Besides, in regard of Comparative Example 41, absorbance at a wavelength of 280 nm was measured as measurement of protein concentration, and, further, the activity of anti-TNFα monoclonal antibody Fab fragment was measured. From the results of these measurements, the ratio of the protein concentration in Example 41 to the protein concentration in Comparative Example 41, and the ratio of the anti-TNFα monoclonal antibody Fab fragment activity in Example 41to the anti-TNFα monoclonal antibody Fab fragment activity in Comparative Example 41 were determined. It was found that both the protein concentration ratio and the activity ratio rose to about 10 times, depicting that the anti-TNFα monoclonal antibody Fab fragment can be concentrated without damaging the activity thereof. The results were set forth in Table 15.

**[Table 15]**

| | | Comparative Example 41 | Example 41 | | |
|---|---|---|---|---|---|
| Test Example 12 | Polyamino acid/protein mass ratio | 0 | 0.05 | 0.1 | 0.3 |
| | Protein concentration ratio | 1 | 9.8 | 8.6 | 4.5 |
| | Activity ratio | 1 | 10.2 | 9.3 | 5.2 |

### [5. Examples 39, 42, 43, and 43-2 in which secondary structure is maintained upon concentration of protein]

### <Anti-TNFα monoclonal antibody>

### (Measurement of CD spectrum of Example 39)

### [Test Example 13]

In regard of Example 39 above, sodium chloride was added to the obtained anti-TNFα monoclonal antibody-poly-L-glutamic acid complex-containing aqueous suspension so as to attain a concentration of 150 mM, and CD spectrum was measured. Besides, a liquid obtained by preparing anti-TNFα monoclonal antibody in a 10 mM MOPS buffer (pH 6.5) so as to attain a concentration of 15 mg/mL was made to be a control liquid (Comparative Example 39), which was put to measurement of CD. As a result, the CD spectra of both the liquids coincided with each other, verifying that the secondary structure of the protein was not changed but maintained upon concentration of the protein. The results were depicted in FIG. 2.

### (Example 42)

In a 10 mM MOPS buffer (pH 7.0), anti-TNFα monoclonal antibody was prepared so as to attain a concentration of 0.5 mg/mL, and poly-L-glutamic acid (MW: 3 kDa to 15 kDa) was added thereto in an amount of 0.04 part by mass based on 1 part by mass of anti-TNFα monoclonal antibody. Each of these prepared liquids was centrifuged, and 90% of the total volume of the centrifuged liquid was removed as supernatant, to obtain an anti-TNFα monoclonal antibody-poly-L-glutamic acid complex-containing aqueous suspension.

### [Test Example 14]

In regard of Example 42 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 5.0 mg/mL. Further, sodium chloride was added to the anti-TNFα monoclonal antibody-poly-L-glutamic acid complex-containing aqueous suspension so as to attain a concentration of 150 mM, and CD spectrum of the resulting liquid was measured. Besides, a liquid obtained by preparing anti-TNFα monoclonal antibody in a 10 mM MOPS buffer (pH 7.0) so as to attain a concentration of 0.5 mg/mL was made to be a control liquid (Comparative Example 42), which was put to measurement of CD spectrum. As a result, the CD spectra of both the liquids coincided with each other, verifying that the secondary structure of the protein was not changed but maintained upon concentration of the protein. The results were depicted in FIG. 3.

### (Example 43)

In a 10 mM citrate buffer (pH 4.7), anti-TNFα monoclonal antibody was prepared so as to attain a concentration of 0.5 mg/mL, and poly-L-glutamic acid (MW: 3 kDa to 15 kDa) was added thereto in an amount of 0.5 part by mass based on 1 part by mass of anti-TNFα monoclonal antibody. Each of these prepared liquids was centrifuged, and 90% of the total volume of the centrifuged liquid was removed as supernatant, to obtain an anti-TNFα monoclonal antibody-poly-L-glutamic acid complex-containing aqueous suspension.

### [Test Example 15]

In regard of Example 43 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 5.0 mg/mL. Further, sodium chloride was added to the anti-TNFα monoclonal antibody-poly-L-glutamic acid complex-containing aqueous suspension so as to attain a concentration of 150 mM, and CD spectrum was measured. Besides, a liquid obtained by preparing anti-TNFα monoclonal antibody in a 10 mM citrate buffer (pH 5.2) so as to attain a concentration of 0.5 mg/mL was made to be a control liquid (Comparative Example 43), which was put to measurement of CD spectrum. As a result, the CD spectra of both of the liquids coincided with each other, verifying that the secondary structure of the protein was not changed but maintained upon concentration of the protein. The results were depicted in FIG. 4.

### <Human IgG>

### (Example 43-2)

In a 10 mM citrate buffer (pH 5.0), human IgG was prepared so as to attain a concentration of 30 mg/mL, and poly-L-glutamic acid (MW: 3 kDa to 15 kDa) was added thereto in an amount of 0.05 part by mass based on 1 part by mass of human IgG. Each of these prepared liquids was centrifuged, and 90% of the total volume of the centrifuged liquid was removed as supernatant, to obtain a human IgG-poly-L-glutamic acid complex-containing aqueous suspension.

### [Test Example 15-2]

In regard of Example 43-2 above, part of the obtained human IgG-poly-L-glutamic acid complex-containing aqueous suspension was sampled, sodium chloride was added thereto so as to attain a concentration of 600 mM, absorbance at a wavelength of 280 nm was measured as measurement of protein concentration, and, further, CD spectrum was measured. Besides, a liquid obtained by preparing human IgG in a 10 mM citrate buffer (pH 5.0) so as to attain a concentration of 0.5 mg/mL was made to be a control liquid (Comparative Example 43-2), which was put to measurement of CD spectrum. As a result, the CD spectra of both of the liquids coincided with each other, verifying that the secondary structure of the protein was not changed but maintained upon concentration of the protein. The results were depicted in FIG. 5.

### [6. Examples 44 to 49 which depict stabilizing effect against shaking stress]

### <Anti-EGFR monoclonal antibody>

### (Example 44)

In a 10 mM MOPS buffer (pH 5.0), anti-EGFR monoclonal antibody was prepared so as to attain a concentration of 13.3 mg/mL, and poly-L-glutamic acid (MW: 3 kDa to 15 kDa) was added thereto in an amount of 0.07 part by mass based on 1 part by mass of anti-EGFR monoclonal antibody. The thus prepared liquid was centrifuged, and 90% of the total volume of the centrifuged liquid was removed as supernatant, to obtain an anti-EGFR monoclonal antibody-poly-L-glutamic acid complex-containing aqueous suspension.

### [Test Example 18]

In regard of Example 44 above, part of the obtained anti-EGFR monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 78.5 mg/mL. The rest of the anti-EGFR monoclonal antibody-poly-L-glutamic acid complex-containing aqueous suspension was filled into a polypropylene-made disposable syringe, which was placed on a shaker (Bioshaker V·BR-36), and shaking at 500 rpm was conducted at room temperature for 90 hours. Sodium chloride was added to the anti-EGFR monoclonal antibody-poly-L-glutamic acid complex-containing aqueous suspension before the shaking and to that after the shaking so as to attain a concentration of 150 mM, the resulting aqueous suspensions were centrifuged at 10,000 g for 10 minutes, and supernatants were put to measurement of absorbance at a wavelength of 280 nm as measurement of protein concentration. Besides, a liquid obtained by preparing anti-EGFR in a 10 mM MOPS buffer (pH 5.0) so as to attain a concentration of 78.5 mg/mL was made to be a control liquid (Comparative Example 44), which was filled into a syringe, and subjected to shaking simultaneously with the aqueous suspension. Sodium chloride was added to the control liquid (Comparative Example 44) before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-EGFR monoclonal antibody-poly-L-glutamic acid complex-containing aqueous suspension and the control liquid. For the control liquid, a lowering in the retention rate was observed. For the anti-EGFR monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 16.

**[Table 16]**

| | | Control liquid (Comparative Example 44) | Protein-polyamino acid complex-containing aqueous suspension (Example 44) | Significant test (t-test) |
|---|---|---|---|---|
| Test Example 18 | Retention rate of protein content after shaking based on protein content before shaking (%) | 86.6 | 105.3 | 0.0008 |

### <Anti-TNFα monoclonal antibody>

### (Example 45)

In a 10 mM MOPS buffer (pH 7.0), anti-TNFα monoclonal antibody was prepared so as to attain a concentration of 0.5 mg/mL, and poly-L-glutamic acid (MW: 3 kDa to 15 kDa) was added thereto in an amount of 0.04 part by mass based on 1 part by mass of anti-TNFα monoclonal antibody. The thus prepared liquid was centrifuged, and 90% of the total volume of the centrifuged liquid was removed as supernatant, to obtain an anti-TNFα monoclonal antibody-poly-L-glutamic acid complex-containing aqueous suspension.

### [Test Example 19]

In regard of Example 45 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 5.0 mg/mL. To the rest of the anti-TNFα monoclonal antibody-poly-L-glutamic acid complex-containing aqueous suspension, a ninefold amount of a 10 mM MOPS buffer (pH 7.0) was added to attain a protein concentration of 0.5 mg/mL. The thus diluted aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-TNFα monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration.

Besides, a liquid obtained by preparing anti-TNFα monoclonal antibody in a 10 mM MOPS buffer (pH 7.0) so as to attain a concentration of 0.5 mg/mL was made to be a control liquid (Comparative Example 45), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-TNFα monoclonal antibody-poly-L-glutamic acid complex-containing aqueous suspension and the control liquid. For the control liquid, a conspicuous lowering in the retention rate was observed. For the anti-TNFα monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 17.

**[Table 17]**

| | | Control liquid (Comparative Example 45) | Protein-polyamino acid complex-containing aqueous suspension (Example 45) |
|---|---|---|---|
| Test Example 19 | Retention rate of protein content after shaking based on protein content before shaking (%) | 6 | 70.0 |

### (Example 46)

In a 10 mM MOPS buffer (pH 6.5), anti-TNFα monoclonal antibody was prepared so as to attain a concentration of 0.5 mg/mL, and poly-L-glutamic acid (MW: 3 kDa to 15 kDa) was added thereto in an amount of 0.05 part by mass based on 1 part by mass of anti-TNFα monoclonal antibody. The thus prepared liquid was centrifuged, and 90% of the total volume of the centrifuged liquid was removed as supernatant, to obtain an anti-TNFα monoclonal antibody-poly-L-glutamic acid complex-containing aqueous suspension.

### [Test Example 20]

In regard of Example 46 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 5.0 mg/mL. To the rest of the anti-TNFα monoclonal antibody-poly-L-glutamic acid complex-containing aqueous suspension, a ninefold amount of a 10 mM MOPS buffer (pH 6.5) was added to attain a protein concentration of 0.5 mg/mL. The thus diluted aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-TNFα monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration.

Besides, a liquid obtained by preparing anti-TNFα monoclonal antibody in a 10 mM MOPS buffer (pH 6.5) so as to attain a concentration of 0.5 mg/mL was made to be a control liquid (Comparative Example 46), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-TNFα monoclonal antibody-poly-L-glutamic acid complex-containing aqueous suspension and the control liquid. For the control liquid, a conspicuous lowering in the retention rate was observed. For the anti-TNFα monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 18.

**[Table 18]**

| | | Control liquid (Comparative Example 46) | Protein-polyamino acid complex-containing aqueous suspension (Example 46) |
|---|---|---|---|
| Test Example 20 | Retention rate of protein content after shaking based on protein content before shaking (%) | 10.6 | 94.9 |

### [Test Example 21]

In regard of Example 46 above, sodium chloride was added to the obtained anti-TNFα monoclonal antibody-poly-L-glutamic acid complex-containing aqueous suspension so as to attain a concentration of 150 mM, and the activity of anti-TNFα monoclonal antibody was measured. Besides, in regard of Comparative Example 46, the activity of anti-TNFα monoclonal antibody was measured. From the results of these measurements, the retention rate of the activity after shaking based on the activity before shaking was determined for the anti-TNFα monoclonal antibody-poly-L-glutamic acid complex-containing aqueous suspension and the control liquid. For the control liquid, a conspicuous lowering in the activity retention rate was observed. For the anti-TNFα monoclonal antibody-poly-L-glutamic acid complex-containing aqueous suspension, on the other hand, a lowering in the activity retention rate was restrained significantly. Thus, a stabilizing effect against shaking stress was depicted as to the protein-polyamino acid complex-containing aqueous suspension. The results were set forth in Table 19.

**[Table 19]**

| | | Control liquid (Comparative Example 46) | Protein-polyamino acid complex-containing aqueous suspension (Example 46) |
|---|---|---|---|
| Test Example 21 | Retention rate of protein content after shaking based on protein content before shaking (%) | 37.5 | 96.8 |

### (Example 47)

In a 10 mM MOPS buffer (pH 6.5), anti-TNFα monoclonal antibody was prepared so as to attain a concentration of 5 mg/mL, and poly-L-glutamic acid (MW: 3 kDa to 15 kDa) was added thereto in an amount of 0.05 part by mass based on 1 part by mass of anti-TNFα monoclonal antibody. The thus prepared liquid was centrifuged, and 90% of the total volume of the centrifuged liquid was removed as supernatant, to obtain an anti-TNFα monoclonal antibody-poly-L-glutamic acid complex-containing aqueous suspension.

### [Test Example 22]

In regard of Example 47 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50.0 mg/mL. To the rest of the anti-TNFα monoclonal antibody-poly-L-glutamic acid complex-containing aqueous suspension, a ninefold amount of a 10 mM MOPS buffer (pH 6.5) was added to attain a protein concentration of 5.0 mg/mL. The thus diluted aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-TNFα monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration.

Besides, a liquid obtained by preparing anti-TNFα monoclonal antibody in a 10 mM MOPS buffer (pH 6.5) so as to attain a concentration of 5.0 mg/mL was made to be a control liquid (Comparative Example 47), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-TNFα monoclonal antibody-poly-L-glutamic acid complex-containing aqueous suspension and the control liquid. For the control liquid, a lowering in the retention rate was observed. For the anti-TNFα monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 20.

**[Table 20]**

| | | Control liquid (Comparative Example 47) | Protein-polyamino acid complex-containing aqueous suspension (Example 47) | Significant test (t-test) |
|---|---|---|---|---|
| Test Example 22 | Retention rate of protein content after shaking based on protein content before shaking (%) | 75.2 | 103.5 | 0.0006 |

### (Example 48)

The same operations as in Example 47 were conducted, except that the amount of the solution prepared was increased, specifically that while 90% of the total volume of the centrifuged liquid was removed as supernatant to obtain 0.06 mL of the anti-TNFα monoclonal antibody-poly-L-glutamic acid complex-containing aqueous suspension in Example 47, the amount of the liquid prepared before the centrifugation was increased to 6/8 times that in Example 47 and, thereby, 0.08 mL of an aqueous suspension was obtained.

### [Test Example 23]

In regard of Example 48 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50.0 mg/mL. To the rest of the anti-TNFα monoclonal antibody-poly-L-glutamic acid complex-containing aqueous suspension, a ninefold amount of a 10 mM MOPS buffer (pH 6.5) was added to attain a protein concentration of 5.0 mg/mL. The thus diluted aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-TNFα monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration.

Besides, a liquid obtained by preparing anti-TNFα monoclonal antibody in a 10 mM MOPS buffer (pH 6.5) so as to attain a concentration of 5.0 mg/mL was made to be a control liquid (Comparative Example 48), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-TNFα monoclonal antibody-poly-L-glutamic acid complex-containing aqueous suspension and the control liquid. For the control liquid, a lowering in the retention rate was observed. For the anti-TNFα monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 21.

**[Table 21]**

| | | Control liquid (Comparative Example 48) | Protein-polyamino acid complex-containing aqueous suspension (Example 48) |
|---|---|---|---|
| Test Example 23 | Retention rate of protein content after shaking based on protein content before shaking (%) | 52.1 | 98.3 |

### [Test Example 24]

In regard of the same sample as the sample which was subjected to measurement of protein concentration in the supernatant in Test Example 23 above, the activity of anti-TNFα monoclonal antibody was measured. From the results of these measurements, the retention rate of the activity after shaking based on the activity before shaking was determined for the anti-TNFα monoclonal antibody-poly-L-glutamic acid complex-containing aqueous suspension and the control liquid. For the control liquid, a conspicuous lowering in the activity retention rate was observed. For the anti-TNFα monoclonal antibody-poly-L-glutamic acid complex-containing aqueous suspension, on the other hand, a lowering in the activity retention rate was restrained significantly. Thus, a stabilizing effect against shaking stress was depicted as to the protein-polyamino acid complex-containing aqueous suspension. The results were set forth in Table 22.

**[Table 22]**

| | | Control liquid (Comparative Example 48) | Protein-polyamino acid complex-containing aqueous suspension (Example 48) |
|---|---|---|---|
| Test Example 24 | Retention rate of protein content after shaking based on protein content before shaking (%) | 53.8 | 102.7 |

### <Anti-IgE monoclonal antibody>

### (Example 49)

In a 10 mM citrate buffer (pH 5.4), anti-IgE monoclonal antibody was prepared so as to attain a concentration of 0.5 mg/mL, and poly-L-glutamic acid (MW: 3 kDa to 15 kDa) was added thereto in an amount of 0.1 part by mass based on 1 part by mass of anti-IgE monoclonal antibody. The thus prepared liquid was centrifuged, and 90% of the total volume of the centrifuged liquid was removed as supernatant, to obtain an anti-IgE monoclonal antibody-poly-L-glutamic acid complex-containing aqueous suspension.

### [Test Example 25]

In regard of Example 49 above, part of the obtained anti-IgE monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 5.0 mg/mL. To the rest of the anti-IgE monoclonal antibody-poly-L-glutamic acid complex-containing aqueous suspension, a ninefold amount of a 10 mM citrate buffer (pH 5.4) was added to attain a protein concentration of 0.5 mg/mL. The thus diluted aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-IgE monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration.

Besides, a liquid obtained by preparing anti-IgE monoclonal antibody in a 10 mM citrate buffer (pH 5.5) so as to attain a concentration of 0.5 mg/mL was made to be a control liquid (Comparative Example 49), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, the retention rate of protein content after the shaking based on the protein content before the shaking was determined, for the anti-IgE monoclonal antibody-poly-L-glutamic acid complex-containing aqueous suspension and the control liquid. For the control liquid, a conspicuous lowering in the retention rate was observed. For the anti-IgE monoclonal antibody-poly-L-glutamic acid complex-containing aqueous suspension, on the other hand, a lowering in the retention rate was not observed. Thus, a stabilizing effect against shaking stress was depicted as to the protein-polyamino acid complex-containing aqueous suspension. The results were set forth in Table 23.

**[Table 23]**

| | | Control liquid (Comparative Example 49) | Protein-polyamino acid complex-containing aqueous suspension (Example 49) |
|---|---|---|---|
| Test Example 25 | Retention rate of protein content after shaking based on protein content before shaking (%) | 9 | 97 |

### [7. Examples 50 and 51 in which secondary structure is maintained against shaking stress]

### <Anti-TNFα monoclonal antibody>

### [Test Example 26]

In regard of the anti-TNFα monoclonal antibody-poly-L-glutamic acid complex-containing aqueous suspension (Example 45) obtained in Test Example 19 above, CD spectrum was measured after the shaking. On the other hand, in regard of the control liquid (Comparative Example 45) obtained in Test Example 19, CD spectrum was measured before the shaking. As a result, the CD spectrum in the case where the shaking stress was exerted on the anti-TNFα monoclonal antibody-poly-L-glutamic acid complex-containing aqueous suspension and the CD spectrum in the case where the shaking stress was not exerted coincided with each other. Thus it was verified that the secondary structure is maintained even when shaking stress is exerted on the anti-TNFα monoclonal antibody-poly-L-glutamic acid complex-containing aqueous suspension. The results are depicted in FIG. 6.

### <Anti-IgE monoclonal antibody>

### [Test Example 27]

In regard of the anti-IgE monoclonal antibody-poly-L-glutamic acid complex-containing aqueous suspension (Example 49) obtained in Test Example 25 above, CD spectrum was measured after the shaking. On the other hand, in regard of the control liquid (Comparative Example 49) obtained in Test Example 25, CD spectrum was measured before the shaking and after the shaking. As a result, the CD spectrum in the case where shaking stress was exerted on the control liquid and the CD spectrum in the case where the shaking stress was not exerted did not coincide with each other, and a change in secondary structure is generated. On the other hand, the CD spectrum in the case where shaking stress was exerted on the anti-IgE monoclonal antibody-poly-L-glutamic acid complex-containing aqueous suspension and the CD spectrum in the case where the shaking stress was not exerted coincided with each other. Thus, it was verified that the secondary structure is maintained even when shaking stress is exerted on the anti-IgE monoclonal antibody-poly-L-glutamic acid complex-containing aqueous suspension. The results were depicted in FIG. 7.

### [8. Example 52 which depicts enhancement of fluidity]

### <Anti-IgE monoclonal antibody>

### (Example 52)

In a 10 mM MOPS buffer (pH 5.5), anti-IgE monoclonal antibody was prepared so as to attain each of concentrations of 6.0 mg/mL, 8.0 mg/mL, and 10.0 mg/mL, and poly-L-glutamic acid (MW: 3 kDa to 15 kDa) was added individually thereto in an amount of 0.08 part by mass based on 1 part by mass of anti-IgE monoclonal antibody. The thus prepared liquids were centrifuged, and 90% of the total volume of the centrifuged liquid was removed as supernatant, to obtain each anti-IgE monoclonal antibody-poly-L-glutamic acid complex-containing aqueous suspension.

### [Test Example 28]

In regard of Example 52 above, part of each of the obtained anti-IgE monoclonal antibody-poly-L-glutamic acid complex-containing aqueous suspensions was sampled, sodium chloride was added thereto so as to attain a concentration of 150 mM, and absorbance at a wavelength of 280 nm was measured as measurements of protein concentration, which were 60.0 mg/mL, 80.0 mg/mL, and 100.0 mg/mL. In regard of the rest of each anti-IgE monoclonal antibody-poly-L-glutamic acid complex-containing aqueous suspension, viscosity was measured by a differential pressure type viscometer. Besides, liquids obtained by preparing anti-IgE monoclonal antibody in a 10 mM MOPS buffer (pH 5.5) so as to attain concentrations of 60.0 mg/mL, 80.0 mg/mL, and 100.0 mg/mL were made to be control liquids (Comparative Example 52), and viscosity was measured by a differential pressure type viscometer. From the results of these measurements, it was verified that the anti-IgE monoclonal antibody-poly-L-glutamic acid complex-containing aqueous suspensions are lower in viscosity and higher in fluidity than the control liquids. The results were set forth in Table 24.

**[Table 24]**

| | Protein concentration (mg/mL) | Viscosity (mPa/s) | |
|---|---|---|---|
| | | Control liquid (Comparative Example 52) | Protein-polyamino acid complex-containing aqueous suspension (Example 52) |
| Test Example 28 | 60 | 4.62 | 2.74 |
| | 80 | 9.6 | 5.2 |
| | 100 | 21.87 | 10.96 |

### [9. Example 53 which depicts stabilizing effect against oxidation stress]

### <Anti-IgE monoclonal antibody>

### (Example 53)

In a 10 mM MOPS buffer (pH 5.5), anti-IgE monoclonal antibody was prepared so as to attain a concentration of 6.0 mg/mL, and poly-L-glutamic acid (MW: 3 kDa to 15 kDa) was added thereto in an amount of 0.1 part by mass based on 1 part by mass of anti-IgE monoclonal antibody. The thus prepared liquid was centrifuged, and 90% of the total volume of the centrifuged liquid was removed as supernatant, to obtain an anti-IgE monoclonal antibody-poly-L-glutamic acid complex-containing aqueous suspension.

### [Test Example 31]

In regard of Example 53 above, part of the obtained anti-IgE monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 60.0 mg/mL. To the rest of the anti-IgE monoclonal antibody-poly-L-glutamic acid complex-containing aqueous suspension, a 0.1-fold amount of 0.6% by weight hydrogen peroxide was added, to obtain anti-IgE monoclonal antibody-poly-L-glutamic acid complex-containing aqueous suspension/0.1% hydrogen peroxide. This liquid was kept at 37°C for two hours. Besides, a 0.1-fold amount of water was added in place of the 0.6% by weight hydrogen peroxide, to obtain a 50.0 mg/mL anti-IgE monoclonal antibody-poly-L-glutamic acid complex-containing aqueous suspension. These liquids were kept at 37°C for two hours, and then sodium chloride was added thereto so as to attain a concentration of 150 mM. Further, the liquids were subjected to fragmentation by trypsin, and the fragmentation products were subjected to analysis of primary structure by a peptide mapping method. Besides, a liquid obtained by preparing anti-IgE monoclonal antibody in a 10 mM MOPS buffer (pH 5.5) so as to attain a concentration of 60.0 mg/mL was made to be a control liquid (Comparative Example 53), and a 0.1-fold amount of 0.6% by weight hydrogen peroxide was added thereto, to obtain 50.0 mg/mL anti-IgE monoclonal antibody/0.1% hydrogen peroxide. Further, a 0.1-fold amount of water was added in place of the 0.6% by weight hydrogen peroxide, to obtain 50.0 mg/mL anti-IgE monoclonal antibody. These control liquids were kept at 37°C for two hours, followed by adding sodium chloride thereto so as to attain a concentration of 150 mM. Further, the liquids were subjected to fragmentation by trypsin, and the fragmentation products were subjected to analysis of primary structure by a peptide mapping method. From the results of these operations, rate of change in primary structure in the presence of hydrogen peroxide based on primary structure in the absence of hydrogen peroxide was calculated from rate of decrease in peak area, for the anti-IgE monoclonal antibody-poly-L-glutamic acid complex-containing aqueous suspension and the control liquids. The rate of change in primary structure in the anti-IgE monoclonal antibody-poly-L-glutamic acid complex-containing aqueous suspension (Example 53) was significantly suppressed, as compared with the rate of change in primary structure in the control liquid (Comparative Example 53). Thus, a stabilizing effect against oxidation stress was depicted as to the protein-polyamino acid complex-containing aqueous suspension. The results, with the rate of change in primary structure in the control liquid being taken as 1, were set forth in Table 25.

**[Table 25]**

| | | Control liquid (Comparative Example 53) | Protein-polyamino acid complex-containing aqueous suspension (Example 53) |
|---|---|---|---|
| Test Example 31 | Rate of change in primary structure (%) | 100 | 86.6 |

### [10. Examples 54 to 55 which depict stabilizing effect against thermal stress]

### (Example 54)

In a 10 mM citrate buffer (pH 5.5), anti-IgE monoclonal antibody was prepared so as to attain a concentration of 5 mg/mL, and poly-L-glutamic acid (MW: 3 kDa to 15 kDa) was added thereto in an amount of 0.05 part by mass based on 1 part by mass of anti-IgE monoclonal antibody. The thus prepared liquid was centrifuged, and 90% of the total volume of the centrifuged liquid was removed as supernatant, to obtain an anti-IgE monoclonal antibody-poly-L-glutamic acid complex-containing aqueous suspension.

### [Test Example 35]

In regard of Example 54 above, part of the obtained anti-IgE monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50 mg/mL. To the rest of the anti-IgE monoclonal antibody-poly-L-glutamic acid complex-containing aqueous suspension, a ninefold amount of a 10 mM citrate buffer (pH 5.5) was added to attain a concentration of 5 mg/mL, then the resulting liquid was kept at 60°C or a cold temperature for 15 hours, or at 50°C or a cold temperature for 60 hours, after which sodium chloride was added so as to attain a concentration of 150 mM, and the activity of anti-IgE monoclonal antibody was measured. Besides, a liquid obtained by preparing anti-IgE monoclonal antibody in a 10 mM citrate buffer (pH 5.5) so as to attain a concentration of 5 mg/mL was made to be a control liquid, which was kept at 60°C or a cold temperature for 15 hours, or at 50°C or a cold temperature for 60 hours, thereafter sodium chloride was added so as to attain a concentration of 150 mM, and the activity of anti-IgE monoclonal antibody was measured. From the results of these measurements, the retention rate of the activity of anti-IgE monoclonal antibody when heated based on the activity of anti-IgE monoclonal antibody when not heated was determined, for the anti-IgE monoclonal antibody-poly-L-glutamic acid complex-containing aqueous suspension and the control liquid. In the control liquid, the activity was lowered. In the anti-IgE monoclonal antibody-poly-L-glutamic acid complex-containing aqueous suspension, on the other hand, the lowering of the activity was restrained. Thus, a stabilizing effect against thermal stress was depicted as to the protein-polyamino acid complex-containing aqueous suspension. The results were set forth in Table 26.

**[Table 26]**

| | Heating conditions | Retention rate of activity when heated based on activity when not heated (%) | |
|---|---|---|---|
| | | Control liquid (Comparative Example 54) | Protein-polyamino acid complex-containing aqueous suspension (Example 54) |
| Test Example 35 | 60°C × 16 hr | 51.8 | 84.3 |
| | 50°C × 60 hr | 43.2 | 86.0 |

### (Example 55)

In a 10 mM citrate buffer (pH 5.4), anti-IgE monoclonal antibody was prepared so as to attain a concentration of 5.0 mg/mL, and poly-L-glutamic acid (MW: 3 kDa to 15 kDa) was added thereto in an amount of 0.075 part by mass based on 1 part by mass of anti-IgE monoclonal antibody. The thus prepared liquid was centrifuged, and 90% of the total volume of the centrifuged liquid was removed as supernatant, to obtain an anti-IgE monoclonal antibody-poly-L-glutamic acid complex-containing aqueous suspension.

### [Test Example 36]

In regard of Example 55 above, part of the obtained anti-IgE monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50.0 mg/mL. The rest of the anti-IgE monoclonal antibody-poly-L-glutamic acid complex-containing aqueous suspension was put to differential scanning calorimetry. Besides, a liquid obtained by preparing anti-IgE monoclonal antibody in a 10 mM citrate buffer (pH 5.4) so as to attain a concentration of 50.0 mg/mL was made to be a control liquid, which was put to differential scanning calorimetry. For each of the aqueous suspension and the control liquid, denaturing temperature was determined. It was made clear that in the anti-IgE monoclonal antibody-poly-L-glutamic acid complex-containing aqueous suspension, denaturation of protein occurs at a high temperature, as compared to the control liquid. Thus, a stabilizing effect against thermal stress was depicted as to the protein-polyamino acid complex-containing aqueous suspension. The results were set forth in Table 27.

**[Table 27]**

| | | Denaturing temperature 1 (°C) | Denaturing temperature 2 (°C) |
|---|---|---|---|
| Test Example 36 | Control liquid (Comparative Example 55) | 71.7 | 83.41 |
| | Protein-polyamino acid complex-containing aqueous suspension (Example 55) | 79.44 | 90 |

### [11. Examples 56 to 58 which depict aggregation inhibitory effect]

### <Anti-EGFR monoclonal antibody>

### (Example 56)

In a 10 mM MOPS buffer (pH 5.0), anti-EGFR monoclonal antibody was prepared so as to attain a concentration of 5.3 mg/mL, and poly-L-glutamic acid (MW: 3 kDa to 15 kDa) was added thereto in an amount of 0.05 part by mass based on 1 part by mass of anti-EGFR monoclonal antibody. The thus prepared liquid was centrifuged, and 94% of the total volume of the centrifuged liquid was removed as supernatant, to obtain an anti-EGFR monoclonal antibody-poly-L-glutamic acid complex-containing aqueous suspension.

### [Test Example 37]

In regard of Example 56 above, part of the obtained anti-EGFR monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 89.0 mg/mL. The rest of the anti-EGFR monoclonal antibody-poly-L-glutamic acid complex-containing aqueous suspension was filled into a polypropylene-made disposable syringe, which was placed on a shaker (Bioshaker V·BR-36), and shaking at 500 rpm was conducted at room temperature for 90 hours. Sodium chloride was added to the anti-EGFR monoclonal antibody-poly-L-glutamic acid complex-containing aqueous suspension before the shaking and to that after the shaking so as to attain a concentration of 150 mM, the resulting aqueous suspensions were centrifuged at 10,000 g for 15 minutes, and the supernatants were put to measurement of absorbance at a wavelength of 280 nm as measurement of protein concentration (Example 56). Besides, a liquid obtained by preparing anti-EGFR monoclonal antibody in a 10 mM MOPS buffer (pH 5.0) so as to attain a concentration of 89.0 mg/mL was made to be a control liquid (Comparative Example 56), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration. From the results of these measurements, for the anti-EGFR monoclonal antibody-poly-L-glutamic acid complex-containing aqueous suspension and the control liquid, the amount of insoluble aggregates of the protein was calculated by subtracting the mass of the protein in the supernatant from the total mass of the protein, and the rate of conversion from protein before shaking into insoluble protein aggregates after shaking was determined. In the anti-EGFR monoclonal antibody-poly-L-glutamic acid complex-containing aqueous suspension, the rate of conversion from protein into insoluble protein aggregates was suppressed, as compared to the control liquid. Thus, an aggregation inhibitory effect of the protein-polyamino acid complex-containing aqueous suspension was depicted. The results were set forth in Table 28.

**[Table 28]**

| | | Control liquid (Comparative Example 56) | Protein-polyamino acid complex-containing aqueous suspension (Example 56) |
|---|---|---|---|
| Test Example 37 | Rate of conversion from protein before shaking into insoluble protein aggregates after shaking (%) | 10.9 | 1.6 |

### (Example 57)

In a 10 mM MOPS buffer (pH 5.0), anti-EGFR monoclonal antibody was prepared so as to attain a concentration of 5.3 mg/mL, and poly-L-glutamic acid (MW: 3 kDa to 15 kDa) was added thereto in an amount of 0.05 part by mass based on 1 part by mass of anti-EGFR monoclonal antibody. The thus prepared liquid was centrifuged, and 95% of the total volume of the centrifuged liquid was removed as supernatant, to obtain anti-EGFR monoclonal antibody-poly-L-glutamic acid complex-containing aqueous suspension.

### [Test Example 38]

In regard of Example 57 above, part of the obtained anti-EGFR monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 114.9 mg/mL. The rest of the anti-EGFR monoclonal antibody-poly-L-glutamic acid complex-containing aqueous suspension was filled into a polypropylene-made disposable syringe, which was placed on a shaker (Bioshaker V·BR-36), and shaking at 500 rpm was conducted at room temperature for 90 hours. Sodium chloride was added to the anti-EGFR monoclonal antibody-poly-L-glutamic acid complex-containing aqueous suspension before the shaking and to that after the shaking so as to attain a concentration of 150 mM, the resulting liquids were centrifuged at 10,000 g for 15 minutes, and the supernatants were put to measurement of absorbance at a wavelength of 280 nm as measurement of protein concentration (Example 57). Besides, a liquid obtained by preparing anti-EGFR monoclonal antibody in a 10 mM MOPS buffer (pH 5.0) so as to attain a concentration of 114.9 mg/mL was made to be a control liquid (Comparative Example 57), which was filled into a syringe, and subjected to shaking simultaneously with the aqueous suspension. Sodium chloride was added to the control liquid before the shaking and to that after the shaking so as to attain a concentration of 150 mM, the resulting liquids were centrifuged at 10,000 g for 15 minutes, and the supernatants were subjected to measurement of absorbance at a wavelength of 280 nm as measurement of protein concentration. From the results of these measurements, for the anti-EGFR monoclonal antibody-poly-L-glutamic acid complex-containing aqueous suspension and the control liquid, the amount of insoluble aggregates of protein was calculated by subtracting the mass of the protein in the supernatant from the total mass of the protein, and the rate of conversion from the protein before the shaking into the insoluble protein aggregates after the shaking was determined. As a result, aggregation of protein was not recognized in the anti-EGFR monoclonal antibody-poly-L-glutamic acid complex-containing aqueous suspension. Thus, an aggregation inhibitory effect was depicted as to the protein-polyamino acid complex-containing aqueous suspension. The results were set forth in Table 29.

**[Table 29]**

| | | Control liquid (Comparative Example 57) | Protein-polyamino acid complex-containing aqueous suspension (Example 57) |
|---|---|---|---|
| Test Example 38 | Rate of conversion from protein before shaking into insoluble protein aggregates after shaking (%) | 12.8 | 0 |

### <Anti-TNFα monoclonal antibody>

### [Test Example 41]

In regard of Example 48 above, part of the obtained anti-TNFα monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50.0 mg/mL. To the rest of the anti-TNFα monoclonal antibody-poly-L-glutamic acid complex-containing aqueous suspension, a ninefold amount of a 10 mM MOPS buffer (pH 6.5) was added to attain a protein concentration of 5.0 mg/mL, the resulting aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-TNFα monoclonal antibody-poly-L-glutamic acid complex-containing aqueous suspension before the shaking and to that after the shaking so as to attain a concentration of 150 mM, and the number of aggregates was measured by a micro-flow imaging (MFI) method. Besides, a liquid obtained by preparing anti-TNFα monoclonal antibody in a 10 mM MOPS buffer (pH 6.5) so as to attain a concentration of 5.0 mg/mL was made to be a control liquid (Comparative Example 48), which was filled into a syringe, and subjected to shaking simultaneously with the aqueous suspension. Sodium chloride was added to the control liquid before the shaking and to that after the shaking so as to attain a concentration of 150 mM, and the number of aggregates was measured by a micro-flow imaging method. From the results of these measurements, for the anti-TNFα monoclonal antibody-poly-L-glutamic acid complex-containing aqueous suspension and the control liquid, the rate of increase in the number of aggregates was determined by dividing the number of aggregates after the shaking by the number of aggregates before the shaking. The rate of increase in the number of aggregates in the anti-TNFα monoclonal antibody-poly-L-glutamic acid complex-containing aqueous suspension was significantly lower than the rate of increase in the number of aggregates in the control liquid. Thus, an aggregation inhibitory effect was depicted as to the protein-polyamino acid complex-containing aqueous suspension. The results were set forth in Table 30.

**[Table 30]**

| | | Control liquid (Comparative Example 48) | Protein-polyamino acid complex-containing aqueous suspension (Example 48) |
|---|---|---|---|
| Test Example 41 | Rate of increase in number of aggregates after shaking based on number of aggregates before shaking (times) | 2121.5 | 2.2 |

### <Anti-IgE monoclonal antibody>

### (Example 58)

In a 10 mM citrate buffer (pH 5.4), anti-IgE monoclonal antibody was prepared so as to attain a concentration of 5.0 mg/mL, and poly-L-glutamic acid (MW: 3 kDa to 15 kDa) was added thereto in an amount of 0.1 part by mass based on 1 part by mass of anti-IgE monoclonal antibody. The thus prepared liquid was centrifuged, and 90% of the total volume of the centrifuged liquid was removes as supernatant, to obtain an anti-IgE monoclonal antibody-poly-L-glutamic acid complex-containing aqueous suspension.

### [Test Example 42]

In regard of Example 58 above, part of the obtained anti-IgE monoclonal antibody-poly-L-glutamic acid complex-containing aqueous suspension was sampled, sodium chloride was added thereto so as to attain a concentration of 150 mM, and absorbance at a wavelength of 280 nm was measured as measurement of protein concentration, which was 50.0 mg/mL. To the rest of the anti-IgE monoclonal antibody-poly-L-glutamic acid complex-containing aqueous suspension, a ninefold amount of a 10 mM citrate buffer (pH 5.4) was added to attain a protein concentration of 5.0 mg/mL, the resulting aqueous suspension was filled into a polypropylene-made disposable syringe, which was placed on a shaker (Bioshaker V·BR-36), and shaking at 500 rpm was conducted at room temperature for 60 hours. Sodium chloride was added to the anti-IgE monoclonal antibody-poly-L-glutamic acid complex-containing aqueous suspension before the shaking and to that after the shaking so as to attain a concentration of 150 mM, and the resulting aqueous suspensions were analyzed by size exclusion chromatography (SEC) (Example 58). Besides, a liquid obtained by preparing anti-IgE monoclonal antibody in a 10 mM citrate buffer (pH 5.4) so as to attain a concentration of 5.0 mg/mL was made to be a control liquid (Comparative Example 58), which was filled into a syringe, and was subjected to shaking simultaneously with the aqueous suspension. Sodium chloride was added to the control liquid before the shaking and to that after the shaking so as to attain a concentration of 150 mM, and the resulting liquids were analyzed by size exclusion chromatography. From the results of these measurements, for the anti-IgE monoclonal antibody-poly-L-glutamic acid complex-containing aqueous suspension and the control liquid, peak area of soluble aggregates was determined. In the control liquid, the soluble aggregate peak area depicted a conspicuous increase. In the anti-IgE monoclonal antibody-poly-L-glutamic acid complex-containing aqueous suspension, on the other hand, the increase of soluble aggregate peak area was significantly suppressed. Thus, an aggregation inhibitory effect was depicted as to the protein-polyamino acid complex-containing aqueous suspension. The results were set forth in Table 31.

**[Table 31]**

| | | Control liquid (Comparative Example 58) | Protein-polyamino acid complex-containing aqueous suspension (Example 58) |
|---|---|---|---|
| Test Example 42 | Rate of increase of soluble aggregate peak area after shaking based on soluble aggregate peak area before shaking (%) | 178.1 | 8.5 |

### [12. Comparative Experiment in which synthetic polymer is used]

### (Control Example 59)

### (1) Confirmation of that anti-TNFα monoclonal antibody can be concentrated using polyacrylic acid

In a 10 mM MOPS buffer (pH 7.0), anti-TNFα monoclonal antibody was prepared so as to attain a concentration of 0.5 mg/mL, and polyacrylic acid (MW: 5 kDa) was added thereto in an amount of 0.04 part by mass based on 1 part by mass of anti-TNFα monoclonal antibody (Control Example 59). Each of the prepared liquids was centrifuged, and 90% of the total volume of the centrifuged liquid was removed as supernatant, to obtain an anti-TNFα monoclonal antibody-polyacrylic acid complex-containing aqueous suspension. Besides, in Comparative Example 59, the same operations as above were conducted, except for not adding polyacrylic acid.

### [Test Example 44]

Sodium chloride was added to this liquid so as to attain a concentration of 150 mM, the resulting liquid was centrifuged, and the resulting supernatant was put to measurement of absorbance at a wavelength of 280 nm as measurement of protein concentration. The results are set forth in Table 44. The protein concentration ratio in Control Example 59 based on the protein concentration in Comparative Example 59 was determined. The protein concentration rose to about 9.9 times. Thus it was depicted that anti-TNFα monoclonal antibody can be concentrated using polyacrylic acid. The results are set forth in Table 32.

**[Table 32]**

| | | Comparative Example 59 | Control Example 59 |
|---|---|---|---|
| Test Example using anti-TNFα monoclonal antibody | Polyacrylic acid/protein mass ratio | 0 | 0.04 |
| | Protein concentration ratio | 1 | 9.9 |

### (2) Confirmation of cytotoxicity of polyglutamic acid and polyacrylic acid

Polyglutamic acid (MW: 3 kDa to 15 kDa), polyacrylic acid (MW: 5 kDa), and polyacrylic acid (MW: 25 kDa) were individually dissolved in a culture medium, and, for each of the polymers, various polymer solutions were thereby prepared so as to attain concentrations of 0% to 1%. Each of the 0% to 1% solutions of each of the polymers was added onto CHO cells seeded on a 96-well plate, and incubation was conducted in a CO2 incubator for 18 hours. After 18 hours, cell growth rate in the case of incubation under each polymer solution was compared with cell growth rate in the case of incubation in a culture medium not containing any polymer (0% polymer solution). As a result, it was depicted that in the case of the polyacrylic acid solution (MW: both 5 kDa and 25 kDa), inhibition of CHO cell growth starts from a concentration of about 0.05% to 0.1%, and the concentration for 50% inhibition of cell growth is around 0.17%. In the case of polyglutamic acid solution, on the other hand, CHO cell growth was little inhibited even with a 1% solution. The results are set forth in Table 33.

**[Table 33]**

| Object polymer | 50% cell growth inhibitory concentration |
|---|---|
| Poly-L-glutamic acid | 1.00% or above |
| Polyacrylic acid (5 kDa) | 0.17% |
| Polyacrylic acid (25 kDa) | 0.17% |

### [13. Confirmation of salt concentration required for re-dissolution of complex]

### <Confirmation of salt concentration required for dissociation of anti-TNFα monoclonal antibody-polyglutamic acid complex>

In a 10 mM MOPS buffer (pH 7.0), anti-TNFα antibody was prepared so as to attain a concentration of 0.5 mg/mL, and poly-L-glutamic acid (MW: 3 kDa to 15 kDa) was added thereto in an amount of 0.05 part by mass based on 1 part by mass of anti-TNFα antibody. Each of these prepared liquids was centrifuged, and 90% of the total volume of the centrifuged liquid was removed as supernatant, to obtain an anti-TNFα monoclonal antibody-poly-L-glutamic acid complex-containing aqueous suspension. To the thus prepared aqueous suspension, a ninefold amount of each of NaCl-containing 10 mM MOPS buffer (pH 7.0) so prepared as to have a NaCl concentration of 0 mM to 100 mM (0% to 0.6% by weight) was added, the resulting liquids were centrifuged, and the supernatants were put to measurement of absorbance at a wavelength of 280 nm as measurement of protein concentration. The ratio of protein concentration in the supernatant after the NaCl addition and centrifugation based on the initial protein concentration was determined. A rise in the protein concentration was confirmed at a NaCl concentration of not less than 10 mM (0.06% by weight). From this result, it was depicted that a NaCl concentration of not less than 10 mM (0.06% by weight) is required for re-dissolution of the anti-TNFα monoclonal antibody-poly-L-glutamic acid complex-containing aqueous suspension, namely, for dissociation of the anti-TNFα monoclonal antibody from the complex. The results are set forth in Table 34.

**[Table 34]**

| | | | | | | | | | | | |
|---|---|---|---|---|---|---|---|---|---|---|---|
| NaCl concentration (mM) | 0 | 2 | 4 | 6 | 8 | 10 | 20 | 40 | 60 | 80 | 100 |
| Protein concentration ratio | 0.1 | 0.1 | 0.1 | 0.1 | 0.1 | 0.2 | 1.8 | 8 | 9.1 | 8.1 | 9.2 |

### [14. Toxicity test of complex-containing aqueous suspension preparation]

In a 10 mM citrate buffer (pH 5.0), rat IgG was prepared so as to attain a concentration of 1 mg/mL, and poly-L-glutamic acid (MW: 50 kDa to 100 kDa) was added thereto in an amount of 0.12 part by mass based on 1 part by mass of rat IgG. The thus prepared liquid was centrifuged, and 90% of the total volume of the centrifuged liquid was removed as supernatant, to obtain a rat IgG-poly-L-glutamic acid complex-containing aqueous suspension. Besides, as a control liquid, rat IgG was prepared in a 10 mM citrate buffer (pH 5.0) so as to attain a concentration of 10 mg/mL. The aqueous suspension and the control liquid were put to subcutaneous injection into a rat's back in a dose of 50 mg/kg. Thereafter, the body weight and the weights of such organs as the heart, lung, liver, spleen, and kidney of the rat were measured. The results are depicted in FIGS. 9(A) and 9(B). Between the control liquid administration group (white circles and white bars) and the complex-containing aqueous suspension administration group (black circles and black bars), no significant difference was found in the body weight or in the weights of the organs (FIGS. 9(A) and 9(B)). Further, pathology examination of the above-mentioned organs and administered parts and blood examination were carried out. All the organs and administered parts were free of any pathological change. Further, between the control liquid administration group and the complex-containing aqueous suspension administration group, there was no significant difference in any of the blood examination items. From these results, it was confirmed that the protein-polyamino acid complex-containing aqueous suspension of the present invention is free of toxicity.

### [15. Confirmation of transition of protein concentration in plasma upon subcutaneous administration of protein-polyamino acid complex-containing aqueous suspension preparation]

In a 10 mM citrate buffer (pH 5.5), anti-IgE monoclonal antibody was prepared so as to attain a concentration of 1 mg/mL, and poly-L-glutamic acid (MW: 50 kDa to 100 kDa) was added thereto in an amount of 0.05 part by mass based on 1 part by mass of anti-IgE monoclonal antibody. Each of these prepared liquids was centrifuged, and 90% of the total volume of the centrifuged liquid was removed as supernatant, to obtain an anti-IgE monoclonal antibody-poly-L-glutamic acid complex-containing aqueous suspension. Besides, as a control liquid, anti-IgE monoclonal antibody was prepared so as to attain a concentration of 10 mg/mL. The aqueous suspension and the control liquid were put to subcutaneous injection into a rat's back in a dose of 10 mg/kg. Thereafter, the concentration of anti-IgE monoclonal antibody in the rat's blood plasma was determined by an ELISA method. The results are depicted in FIG. 10. The plasma anti-IgE monoclonal antibody concentration-time curve for the control liquid administration group (white circles) and that for the complex-containing aqueous suspension administration group (black circles) coincided with each other. Thus, it was confirmed that the control liquid and the aqueous suspension exhibit the same behavior after subcutaneous injection.

### Industrial Applicability

The present invention is high in usefulness as it has one or some of the following characteristics.

The aqueous suspension preparation obtained by the method of the invention is stable against vibration during transportation and against thermal stress or oxidation stress during storage, in regard of proteins as defined above. The present invention forms a complex, whereby stabilization is achieved, and excellent stability during transportation and during storage is ensured. The complex in the present invention can concentrate a protein concentration to a high concentration easily, without need for special equipment such as one for ultrafiltration. A lowering in activity due to concentration is little. The aqueous suspension preparation obtained by the method of the present invention can stabilize a particular protein easily by only selecting the pH of a buffer and an anionic polyamino acid according to the isoelectric point of the protein, without needing addition of an additive or additives which has been conventionally needed. The complex-containing aqueous suspension can be administered as it is as a preparation, without needing a complicated dissolving operation that is necessary in the case of freeze-dried preparations. Alternatively, the complex-containing aqueous suspension can be administered as an aqueous liquid by re-dissolving it through addition of an inorganic salt represented by sodium chloride when put to use. The aqueous suspension preparation obtained by the method of the present invention has a characteristic feature of being low in viscosity even when the protein is present at a high concentration, so that the amount of the aqueous suspension preparation left in a container and wasted at the time of use thereof can be reduced; besides, when administered by use of a syringe, the aqueous suspension preparation can be administered with a weak force, as compared with an aqueous protein solution of the same concentration.

### Description of Reference Symbols

- 1: Prefilled syringe
- 2: Sheath
- 3, 4: Gasket
- 5: Plunger
- 6: Protein-polyamino acid complex
- 7: Dissolving liquid (NaCl solution)
- 8: Enlarged diameter portion

## Claims

1. A method of stabilizing a protein comprising the steps of:
providing a buffer comprising a protein which has a surface charge and
adding a polyamino acid to the buffer to form a complex comprising the protein and the polyamino acid as an aqueous suspension preparation,
wherein the protein in the complex has at least one of shaking stress resistance, fluidity enhancement, oxidation resistance, thermal stability, and aggregation inhibitory properties improved over an aqueous solution of the same protein dissolved in the same buffer and having the same concentration,
the polyamino acid is an anionic polyamino acid which is at least one selected from group consisting of polyglutamic acid, polyaspartic acid and their water-soluble salts,
the protein is at least one selected from group consisting of anti-IgE monoclonal antibody, anti-TNFα monoclonal antibody, anti-TNFα monoclonal antibody Fab fragment, and anti-EGFR monoclonal antibody.

2. The method according to claim 1, further adding a low concentration electrolyte solution to re-dissolve the complex.

3. The method according to claim 1 or 2, wherein a molecular weight of the anionic polyamino acid is in a range from 0.5 kDa to 1000 kDa.

4. The method according to any one of claims 1 to 3, wherein a molecular weight of the protein is in a range from 3 kDa to 670 kDa.

5. The method according to any one of claims 1 to 4, wherein the complex can be concentrated in such a manner that a maximum concentration factor of the protein is present where an absolute value of a difference between pH of the buffer and an isoelectric point (pI) of the protein is in a range from 0.5 to 4.0.

6. The method according to any one of claims 1 to 5, wherein the aqueous suspension preparation having the shaking stress resistance has the complex being high in shaking stress resistance as compared to an aqueous solution of the same protein present at the same concentration in the same buffer.

7. The method according to any one of claims 1 to 6, wherein the aqueous suspension preparation contains no additive other than the buffer, the protein, the polyamino acid and, a pH adjuster.

8. The method according to any one of claims 1 to 7, wherein a secondary structure of the protein is maintained during a process of forming the complex and then solubilizing the complex.

9. An administrating device comprising the protein stabilized by the method according to any one of claims 1 to 8.

## Patentansprüche

1. Verfahren zur Stabilisierung eines Proteins, umfassend die folgenden Schritte:
Bereitstellen eines Puffers, der ein Protein enthält, das eine Oberflächenladung aufweist, und
Hinzufügen einer Polyaminosäure zu dem Puffer, um einen Komplex zu bilden, der das Protein und die Polyaminosäure als eine wässrige Suspensionszubereitung umfasst,
wobei das Protein in dem Komplex mindestens eine der folgenden Eigenschaften aus Schüttelbelastungsbeständigkeit, verbesserter Fließfähigkeit, Oxidationsbeständigkeit, thermischer Stabilität und aggregationshemmenden Eigenschaften aufweist, die gegenüber einer wässrigen Lösung desselben Proteins, das in demselben Puffer gelöst ist und dieselbe Konzentration aufweist, verbessert sind,
wobei die Polyaminosäure eine anionische Polyaminosäure ist, die mindestens eine ist, welche ausgewählt ist aus der Gruppe, bestehend aus Polyglutaminsäure, Polyasparaginsäure und deren wasserlöslichen Salzen,
wobei das Protein mindestens eines ist, welches aus der Gruppe ausgewählt ist, die aus einem monoklonalen Anti-IgE-Antikörper, einem monoklonalen Anti-TNFa-Antikörper, einem Fab-Fragment eines monoklonalen Anti-TNFα-Antikörpers und einem monoklonalen Anti-EGFR-Antikörper besteht.

2. Verfahren nach Anspruch 1, wobei ferner eine Elektrolytlösung mit niedriger Konzentration zugegeben wird, um den Komplex erneut aufzulösen.

3. Verfahren nach Anspruch 1 oder 2, wobei das Molekulargewicht der anionischen Polyaminosäure in einem Bereich von 0,5 kDa bis 1.000 kDa liegt.

4. Verfahren nach einem der Ansprüche 1 bis 3, wobei ein Molekulargewicht des Proteins in einem Bereich von 3 kDa bis 670 kDa liegt.

5. Verfahren nach einem der Ansprüche 1 bis 4, wobei der Komplex in einer derartigen Weise konzentriert werden kann, dass ein maximaler Konzentrationsfaktor des Proteins vorliegt, wobei ein Absolutwert einer Differenz zwischen dem pH-Wert des Puffers und einem isoelektrischen Punkt (pI) des Proteins in einem Bereich von 0,5 bis 4,0 liegt.

6. Verfahren nach einem der Ansprüche 1 bis 5, wobei die wässrige Suspensionszubereitung, die eine hohe Schüttelbelastungsbeständigkeit aufweist, den Komplex enthält, der im Vergleich zu einer wässrigen Lösung desselben Proteins, das in derselben Konzentration in demselben Puffer vorliegt, eine hohe Schüttelbelastungsbeständigkeit aufweist.

7. Verfahren nach einem der Ansprüche 1 bis 6, wobei die wässrige Suspensionszubereitung außer dem Puffer, dem Protein, der Polyaminosäure und einem pH-Einstellmittel keine weiteren Zusatzstoffe enthält.

8. Verfahren nach einem der Ansprüche 1 bis 7, wobei eine Sekundärstruktur des Proteins während eines Prozesses der Bildung des Komplexes und der anschließenden Solubilisierung des Komplexes erhalten bleibt.

9. Vorrichtung zum Verabreichen, umfassend das Protein, das durch das Verfahren nach einem der Ansprüche 1 bis 8 stabilisiert wurde.

## Revendications

1. Procédé de stabilisation d'une protéine comprenant les étapes consistant à :
fournir un tampon comprenant une protéine qui a une charge de surface et
ajouter un polyacide aminé au tampon pour former un complexe comprenant la protéine et le polyacide aminé en tant que préparation en suspension aqueuse,
dans lequel la protéine dans le complexe présente au moins l'une parmi des propriétés de résistance aux stress d'agitation, d'amélioration de fluidité, de résistance à l'oxydation, de stabilité thermique et d'inhibition d'agrégation améliorées par rapport à une solution aqueuse constituée de la même protéine dissoute dans le même tampon et ayant la même concentration,
le polyacide aminé est un polyacide aminé anionique qui est au moins l'un sélectionné parmi le groupe consistant en de l'acide polyglutamique, de l'acide polyaspartique et leurs sels solubles dans l'eau,
la protéine est au moins l'une sélectionnée parmi le groupe consistant en un anticorps monoclonal anti-IgE, un anticorps monoclonal anti-TNFα, un fragment Fab d'anticorps monoclonal anti-TNFα et un anticorps monoclonal anti-EGFR.

2. Procédé selon la revendication 1, comprenant en outre l'ajout d'une solution électrolytique de faible concentration pour re-dissoudre le complexe.

3. Procédé selon la revendication 1 ou 2, dans lequel un poids moléculaire du polyacide aminé anionique est dans une plage de 0,5 kDa à 1 000 kDa.

4. Procédé selon l'une quelconque des revendications 1 à 3, dans lequel un poids moléculaire de la protéine est dans une plage de 3 kDa à 670 kDa.

5. Procédé selon l'une quelconque des revendications 1 à 4, dans lequel le complexe peut être concentré de telle sorte qu'un facteur de concentration maximum de la protéine est présent lorsqu'une valeur absolue d'une différence entre le pH du tampon et un point isoélectrique (pI) de la protéine est dans une plage de 0,5 à 4,0.

6. Procédé selon l'une quelconque des revendications 1 à 5, dans lequel la préparation en suspension aqueuse ayant la résistance au stress d'agitation a le complexe qui est élevé en termes de résistance au stress d'agitation par comparaison à une solution aqueuse de la même protéine présente à la même concentration dans le même tampon.

7. Procédé selon l'une quelconque des revendications 1 à 6, dans lequel la préparation en suspension aqueuse ne contient aucun additif autre que le tampon, la protéine, le polyacide aminé, et un régulateur de pH.

8. Procédé selon l'une quelconque des revendications 1 à 7, dans lequel une structure secondaire de la protéine est conservée pendant un processus de formation du complexe puis de solubilisation du complexe.

9. Dispositif d'administration comprenant la protéine stabilisée par le procédé selon l'une quelconque des revendications 1 à 8.
